# EUROPEAN PATENT SPECIFICATION

(11) **EP 4 081 534 B1**
(45) Date of publication and mention of the grant of the patent: **28.06.2023**
(21) Application number: 21798731.2
(22) Date of filing: 03.11.2021
(51) Int. Cl.: C07K 14/31

(54) **PROTECTIVE STAPHYLOCOCCAL EXOTOXIN VACCINE**
IMPFSTOFF ZUM SCHUTZ GEGEN STAPHYLOKOKKEN-EXOTOXIN
VACCIN DE PROTECTION CONTRE LES EXOTOXINES STAPHYLOCOCCIQUES

(30) Priority: 18.12.2020 EP 20215489
(43) Date of publication of application: 02.11.2022
(73) Proprietor: Biomedizinische Forschung & Bio-Produkte AG, 1090 Wien (AT)
(72) Inventor: EIBL, Martha M., 1090 Wien (AT); MODEL, Nina, 1090 Wien (AT); HALLER, Günter, 1090 Wien (AT)
(74) Representative: Redl, Gerda
(86) International application number: PCT/EP2021/080540
(87) International publication number: WO 2022/128243

(56) References cited:
- WO-A1-99/40935
- WO-A1-2014/205111
- JP-B2- 4 571 586
- WOODY M A ET AL: "Staphylococcal enterotoxin B mutants (N23K and F44S): biological effects and vaccine potential in a mouse model", VACCINE, ELSEVIER, AMSTERDAM, NL, vol. 15, no. 2, 1 February 1997 (1997-02-01), pages 133-139, XP004054362, ISSN: 0264-410X, DOI: 10.1016/S0264-410X(96)00166-1 cited in the application

## Description

### FIELD OF THE INVENTION

The invention relates to a protective Staphylococcal exotoxin vaccine. The present invention particularly relates to novel vaccine antigens and vaccines inducing protective immunity against Staphylococcus aureus infections or exotoxin exposure and protection against a Staphylococcal exotoxin-induced sepsis condition.

### BACKGROUND OF THE INVENTION

Staphylococcus aureus produces a variety of exoproteins that contribute to its ability to colonize and cause disease of varying severity in mammalian hosts ranging from superficial skin infections, such as abscesses and impetigo, to serious invasive infections such as pulmonary disease, osteomyelitis and endocarditis, as well as to the acute and potentially fatal toxic shock syndrome (TSS) and septic shock syndrome.

Staphylococcus aureus expressed exotoxins known as pyrogenic toxin superantigens. Staphylococcal Enterotoxin B is one of the superantigen exotoxins of staphylococci, also understood as Staphylococcal Exotoxin B (SEB). Unlike conventional antigens, superantigens bind to major histocompatibility complex (MHC) class II molecules outside the peptide binding groove and activate T-cells by binding to the Vβ chain of the T cell receptor (TCR). Superantigens are far more potent than conventional antigens and may trigger up to 30% of the entire T cell population. They cause massive proliferation of T cells and uncontrolled release of cytokines. Superantigens activate T-cells at much lower concentrations than nominal antigens. Activation by superantigens can be detected at picogram to low nanogram concentrations, by nominal antigens at micrograms and higher. The difference, thus, is 5 to 8 log steps. Clonal expansion and upregulation of the IL-2 receptor on the cell surface are consequences of cross-linking of MHC class II on antigen presenting cells and TCR on CD4⁺ cells. The massive release of cytokines is the basis of toxicity of SEB. SEB is also a common cause of food poisoning, causing severe diarrhoea, nausea and intestinal cramping. Being quite stable, the toxin may remain active even after contaminating bacteria are killed.

The SEB plays a pivotal role as a causative agent in TSS (Toxic Shock Syndrome). Experimental application of superantigen leads to an overwhelming inflammatory response with uncontrolled release of inflammatory cytokines resulting in shock and multi organ failure (Ulrich et al. Vaccine 1998, 16(19):1857-1864).

Based on information on the structure and functional relationship of S. *aureus* SEB, multiple mutants have been established with the objective of eradicating the toxic and superantigenic properties, while leaving specific immunogenicity and protectivity unimpaired (Lowell et al. Infection and Immunity 1996, 64(5):1706-1713; LeClaire et al. Infection and Immunity 2002, 770(5):2278-2281).

Kappler et al. (J. Exp. Med. 1992, 175:387-396) describe mutations defining functional regions of the superantigen SEB.

Fries et al. (Microbiol Spectr. 2013,1(2): 2013) disclose specific binding regions of SEB and provides a sequence alignment of amino acid sequences of SEB derived from S. *aureus* clinical isolates.

US6713284 discloses genetically attenuated superantigen toxin vaccines, and in particular an SEB wherein certain amino acid positions have been altered such that binding to the MHC Class II receptor and T cell antigen receptor is altered.

Woody et al. (Vaccine 1997, 15(2):133-139) disclose SEB mutants with either N23K or F44S mutations, and describes the vaccine potential in a mouse model.

Jeong et al. (Acta Cryst. 2017, F73, 595-600) disclose an SEB vaccine candidate with four mutations (N23A, Y90A, R110A, and F177A) showing eliminated superantigen activity.

Bagnoli et al. (PNAS 2015, 112(12):3680-3685) describe combined vaccine antigens α-hemolysin (Hla), ess extracellular A (EsxA), and ess extracellular B (EsxB) and the two surface proteins ferric hydroxamate uptake D2 and conserved staphylococcal antigen 1A, formulated with aluminum hydroxide or with a toll-like receptor 7-dependent (TLR7) agonist (SMIP.7-10) adsorbed to alum.

JP4571586B2 (corresponding to EP1661911) discloses a modified SEB being protease-resistant with a reduced toxicity, comprising a substitution of lysine at position 97, and a substitution of lysine at position 98, with any other amino acid. In addition, a substitution of asparagine at position 23 to tyrosine is disclosed.

WO99/40935 (corresponding to EP1055429) discloses an SEB comprising a modification which is one or more amino acid substitutions e.g., at position 9, 23, 41, or 44, to confer inhibitory activity on T cell activation.

WO2014/205111 discloses a multivalent peptide oligopeptide including a *Staphylococcus aureus* antigen or mutant, fragment, variant, or derivative thereof, which may include, SEB, SECI-3, SEE, SEH, SEI, SEK, TSST-1, SpeC, SED, or SpeA, or any mutant, fragment, variant, or derivative thereof, or any combination thereof, in any order. In certain aspects, the oligopeptide is disclosed to include a SEB mutant which is the attenuated toxoid SEB comprising the amino acid substitutions L45R/Y89A/Y94A.

### SUMMARY OF THE INVENTION

It is the objective of the present invention to provide new SEB vaccine antigens to trigger a protective immune response. The objective is solved by the subject of the present claims and as further described herein.

The invention is set out in the appended claims.

The invention provides for a vaccine antigen which confers protective immunity, in particular targeting professional antigen presenting cells (APC) or cells of the innate and/or adaptive immune system expressing constitutively MHC Class II.

The invention provides for a detoxified Staphylococcal Exotoxin B (SEB) toxin that is mutated to comprise three point mutations at amino acid positions 21 to 25 in the SEB toxin sequence SEQ ID NO:1, wherein said three point mutations comprise a deletion of aa21-23 or aa22-24, or a deletion at corresponding amino acid positions in any other naturally-occurring SEB toxin sequence that has at least 95% sequence identity to SEQ ID NO:1.

The invention further provides for a protective vaccine antigen comprising at least one molecule of the detoxified SEB toxin, a protective vaccine, and the use of such vaccine for medical purposes.

The detoxified SEB toxin described herein is also understood as a detoxified SEB or detoxified Staphylococcal exotoxin.

According to a specific aspect, the invention provides for a detoxified Staphylococcal Exotoxin B (SEB) toxin that is mutated to comprise three point mutations at amino acid positions 21 to 25 (i.e., "within aa21-25", also referred to as "in the region of aa21-25") in the SEB toxin sequence SEQ ID NO:1, wherein said three point mutations comprise a deletion of aa21-23 or aa22-24, or at corresponding amino acid positions in any other naturally-occurring SEB toxin sequence that has at least 95%, 96%, 97%, 98% 99%, or 99.5% sequence identity to SEQ ID NO:1. Specifically, such naturally-occurring SEB toxin is functional as a staphylococcal superantigen, unless being engineered to incorporate the respective detoxifying mutations.

Specifically, the wild-type SEB toxin identified by SEQ ID NO:1 originates from S. *aureus* (ATCC 14458). Other naturally-occurring SEB toxin sequences originate from different S. *aureus* sources (or isolates), with variability in regions other than T cell receptor (α/β) binding region (aa21-25 in SEQ ID NO:1; "MENMK", SEQ ID NO:37; identifying positions M21, E22, N23, M24, and K25) or the immunoglobulin superfamily binding region such as e.g. the MHC Class II receptor binding region, in particular the region binding inside, adjacent or outside the antigen-binding groove, (e.g. aa42-47 in SEQ ID NO:1; "DQFLYF", SEQ ID NO:38; identifying positions D42, Q43, F44, L45, Y46, and F47; or aa89-91 in SEQ ID NO:1; or aa66-68 in SEQ ID NO:1). Such regions are herein referred to as "core region". Specifically, either or both of aa21-25 and aa42-47, and optionally any one or both of aa89-91 or aa66-68. Any other regions within the SEB toxin sequence are herein referred to as "non-core regions" of SEB.

Exemplary naturally-occurring SEB wild-type variants comprise or consist of SEQ ID NOs: 17, 19, 21, 23, 25, 27, or 29, each comprising the same core regions, and variability in the non-core regions with at least at least 95%, 96%, 97%, 98% 99%, or 99.5% sequence identity. The number of point mutations within the non-core regions of naturally-occurring SEB wild-type variants of SEQ ID NO:1 may be 1, 2, 3, 4, or 5.

The detoxified SEB toxin is specifically characterized by the detoxifying point mutations in the T cell receptor binding region and optionally in the immunoglobulin superfamily binding region, as further described herein.

Specifically, the detoxifying point mutations are within the T cell receptor binding region of the SEB, more specifically within aa21-25. Specifically, the detoxifying point mutations are in the region of aa21-25, and a deletion of three amino acids. Specifically, the detoxifying point mutations in the region of aa21-25 consist of a deletion of only 3amino acids, preferably at least comprising a deletion of N23 and a deletion of both amino acids which are adjacent to N23. The number of point mutations within aa21-25 can be 3, specifically wherein point mutations are of at least 3 consecutive (adjacent) aa positions, such as aa21-23 or aa22-24.

According to a specific aspect, the mutation in the region of aa21-25 is a deletion of three or four amino acids, at least including a deletion of N23 and E22, and any one of M24or M21, such as a deletion of at least any one of aa21-23 or aa22-24, in particular a deletion of aa22-24 only.

According to a specifically preferred aspect, said point mutations at amino acid positions 21 to 25 comprise or consist of a deletion of amino acids 22-24 or 21-23.

Specifically, the detoxified SEB toxin comprises one or more further point mutations comprising at least one amino acid substitution at a position selected from the group consisting L45, Q43, or F44, preferably L45R, Q43P, F44P, or F44S, preferably wherein the detoxified SEB toxin comprises or consists of any one of SEQ ID NO:5, 7, 9, or 11, or a detoxified SEB variant sequence of any one of the foregoing comprising said three point mutations at amino acid positions 21 to 25 and at least 95% sequence identity to of any one of the foregoing.

According to a specific aspect, the detoxified SEB toxin is characterized by one or more further (additional) point mutations (i.e., other than those in the region of aa21-25) in its immunoglobulin superfamily binding region, preferably in the region of aa42-47, wherein said one or more further point mutations comprise at least one amino acid substitution at a position selected from the group consisting L45, Q43, or F44, preferably L45R, Q43P, F44P, or F44S, preferably at position L45, such as L45R.

In general, mutations made or identified in a sequence (e.g., an amino acid sequence as described herein) are numbered in relation to a reference (or wild-type) sequence, i.e., a sequence that does not contain the mutations. Therefore, unless explicitly described otherwise, the positions of amino acids in the detoxified SEB toxin are those of the respective wild-type toxin e.g. identified by SEQ ID NO:1. Although the detoxified SEB toxin may comprise a deletion of one or more amino acids in the T cell receptor binding region, the positions following any such deletion will still be numbered as in the wild-type protein. For example, in a deletion mutant, deleting aa22-24, the point mutation at L45 is still at a position numbered aa45, though the sequence of such deletion mutant has been shortened by three amino acids.

According to a specific aspect, the detoxified SEB toxin comprises or consists of SEQ ID NO:3, or a detoxified SEB variant sequence thereof comprising said three point mutations at amino acid positions 21 to 25 and at least 95%, 96%, 97%, 98%, 99%, or 99.5% sequence identity to SEQ ID NO:3.

Specifically, the detoxified SEB toxin comprises or consists of SEQ ID NO:3 which differs from SEQ ID NO:1 in the deletion of aa22-24, or a detoxified SEB variant sequence thereof (i.e., a variant SEB sequence of SEQ ID NO:3) comprising said deletion of aa22-24, and at least 95% sequence identity to SEQ ID NO:3.

Specifically, said detoxified SEB variant sequence of SEQ ID NO:3 comprises said deletion of aa22-24 comprised in SEQ ID NO:3; and:
a) one or more further point mutations in its immunoglobulin superfamily binding region, preferably within (i.e., in the region of) aa42-47, and/or
b) one or more further point mutations as naturally-occurring in other regions of the SEB sequence, such as in the non-core regions e.g., one or more point mutations as naturally-occurring in SEB wild-type variants, such as occurring in any one of SEQ ID NO:17, 19, 21, 23, 25, 27, or 29, in particular one or more of the following point mutations: K7N, S14A, V82L, T133S, K141E, T150I, S225F; and/or
c) at least 95%, 96%, 97%, 98% 99%, or 99.5% sequence identity to SEQ ID NO:3, or to the corresponding non-core regions of SEQ ID NO:3.

Specific embodiments comprise a SEB toxin variant sequence of SEQ ID NO:3, which comprises the detoxifying mutations as described herein, and which is particularly characterized by at least any one of 95%, 96%, 97%, 98%, 99%, or 99.5% sequence identity to the respective regions of SEQ ID NO:3, in particular over the whole length of all corresponding regions of SEQ ID NO:3.

Specific embodiments comprise a SEB toxin variant sequence of SEQ ID NO:3, which comprises the detoxifying mutations as described herein, and which is particularly characterized by at least any one of 95%, 96%, 97%, 98%, 99%, or 99.5% sequence identity to the respective non-core regions, in particular over the whole length of all corresponding non-core regions, compared to the corresponding regions of SEQ ID NO:3.

According to specific embodiments, the detoxified SEB toxin comprises or consists of any one of SEQ ID NO:5, 7, 9, or 11, or a detoxified SEB variant sequence of any of the foregoing comprising said three point mutations at amino acid positions 21 to 25 and at least 95%, 96%, 97%, 98%, 99%, or 99.5% sequence identity.

Specifically, said detoxified SEB variant sequence of any one of SEQ ID NO:5, 7, 9, or 11 comprises said at least two or at least three point mutations comprised in the respective SEQ ID NO:5, 7, 9, or 11 at amino acid positions 21 to 25; and:
a) one or more further point mutations in its immunoglobulin superfamily binding region, preferably within aa42-47, and/or
b) one or more further point mutations as naturally-occurring in other regions of the SEB sequence, such as in the non-core regions e.g., one or more point mutations as naturally-occurring in SEB wild-type variants, such as occurring in any one of SEQ ID NO:17, 19, 21, 23, 25, 27, or 29, in particular one or more of the following point mutations: K7N, S14A, V82L, T133S, K141E, T150I, S225F; and/or
c) at least 95%, 96%, 97%, 98%, 99%, or 99.5% sequence identity to the respective SEQ ID NO:5, 7, 9, or 11, or to the corresponding non-core regions of the respective SEQ ID NO:5, 7, 9, or 11.

Specific embodiments comprise a detoxified SEB variant sequence of any one of SEQ ID NO:5, 7, 9, or 11, which comprises the detoxifying mutations as described herein, and which is particularly characterized by at least any one of 95%, 96%, 97%, 98%,99%, or 99.5% sequence identity to the respective non-core regions of any one of the foregoing detoxified SEB toxin sequences, in particular over the whole length of all corresponding non-core regions of any one of the foregoing detoxified SEB toxin sequences.

Specific embodiments comprise a detoxified SEB variant sequence of any one of SEQ ID NO:5, 7, 9, or 11, which comprises the detoxifying mutations as described herein, and which is particularly characterized by at least any one of 95%, 96%, 97%, 98%, 99%, or 99.5% sequence identity to the respective regions of any one of the foregoing detoxified SEB toxin sequences, in particular over the whole length of all corresponding non-core regions of any one of the foregoing detoxified SEB toxin sequences.

Despite of more than one point mutations in the T cell receptor binding region, the SEB toxin described herein has turned out to be not only detoxified, but surprisingly triggers an effective immune response when being used as a vaccine antigen. Such immune response turned out to be protective, and was able to target and prevent the wild-type SEB's ability to bind the T cell receptor, thereby preventing a respective immune reaction and inflammatory response upon challenging with a wild-type SEB and an LPS trigger of the inflammatory immune response.

Therefore, the invention further provides for a protective vaccine antigen comprising or consisting of at least one molecule of the detoxified SEB toxin as described herein.

Specifically, the vaccine antigen comprises or consists of said at least one detoxified SEB toxin molecule in combination with a Staphylococcal vaccine antigen that is identical to or different from said detoxified SEB toxin molecule, preferably wherein the combination is provided as a fusion, mixture, or a complex comprising said molecule and antigen bound to each other and/or bound to a carrier, thereby forming the complex.

Exemplary combinations are fusions of atoxic or detoxified SEB molecules, wherein at least one (or two) of said SEB molecules is the detoxified SEB comprising the point mutations as described herein.

Specifically, said at least one detoxified SEB toxin molecule is fused to a another detoxified SEB toxin molecule, optionally comprising a linker sequence. Specific linkers suitably used are peptidic linkers such as consisting of a series of at least two, or at least three amino acids. Specifically, the fusion of molecules is in any order, by a peptide bond, with or without a linker.

According to a specific aspect, the fusion comprises at least two identical molecules of said detoxified SEB toxin as described herein, or at least two molecules of said detoxified SEB toxin as described herein that differ in at least one of the detoxifying point mutations as described herein. A fusion of at least two molecules, in particular two identical molecules, with or without a linker, is herein also referred as a "tandem construct".

Fusion may be achieved by recombination of nucleic acid molecules encoding the respective peptide sequences, or otherwise by synthesizing the coding nucleic acid molecules or fused (poly)peptide sequences.

Specifically, the linker can be a peptidic linker, preferably composed of a number of consecutive amino acid residues, such as selected from any of the naturally-occurring amino acids, preferably any of Gly, Ser, His, Met, Lys, Leu, and Thr. Linkers can be composed of flexible residues like glycine and serine so that the adjacent peptides are free to move relative to one another. To fuse two detoxified SEB toxin molecules as described herein, short linkers are sufficient, such as a peptide linker comprising or consisting of a sequence of 2-10 amino acids, e.g., comprising or composed of two amino acids selected from the group consisting of Lys-Leu, His-Met, Gly-Thr and Gly-Gly-Gly. Longer linkers such as longer than 10 amino acids can be used e.g., when necessary to ensure that two adjacent molecules do not sterically interfere with each other.

According to a specific aspect, the antigen may comprise one or more peptide spacers in addition to linker, such as to improve the structure or stability of the polypeptide.

Specifically, the peptide fusion described herein may comprise the peptides which are conveniently bound to each other by bioconjugation, chemical conjugation or cross-linking. For example, the antigen may comprise peptide conjugates. Specific embodiments may employ multimerization domains, carriers, or devices such as nanostructures or beads that are suitably used to immobilize a series of peptides.

Yet, the antigen may be provided as a molecule or molecule complex composed of two or more polypeptide chains, which are associated through covalent or noncovalent linkage, or just mixed to obtain an antigenic composition.

According to a specific embodiment, the vaccine antigen comprises or consists of any one of SEQ ID NO:13, 31, 33, or 35, or a vaccine antigen variant sequence of any one of the foregoing comprising said at least two point mutations at amino acid positions 21 to 25 and at least 95%, 96%, 97%, 98%, 99%, or 99.5% sequence identity.

Specifically, said vaccine antigen variant sequence of any one of SEQ ID NO:13, 31, 33, or 35 comprises said at least two or at least three point mutations comprised in the respective SEQ ID NO:13, 31, 33, or 35 at amino acid positions 21 to 25; and:
a) one or more further point mutations in its immunoglobulin superfamily binding region, preferably within aa42-47, and/or
b) one or more further point mutations as naturally-occurring in other regions of the SEB sequence, such as in the non-core regions e.g., one or more point mutations as naturally-occurring in SEB wild-type variants, such as occurring in any one of SEQ ID NO:17, 19, 21, 23, 25, 27, or 29, in particular one or more of the following point mutations: K7N, S14A, V82L, T133S, K141E, T150I, S225F; and/or
c) at least 95%, 96%, 97%, 98%, 99%, or 99.5% sequence identity to the respective SEQ ID NO:13, 31, 33, or 35, or to the corresponding non-core regions of the respective SEQ ID NO:13, 31, 33, or 35.

Specific embodiments comprise a vaccine antigen variant sequence of SEQ ID SEQ ID NO:13, 31, 33, or 35, which comprises the detoxifying mutations as described herein, and which is particularly characterized by at least any one of 95%, 96%, 97%, 98%, 99%, or 99.5% sequence identity to the respective non-core regions of any one of the foregoing sequences, in particular over the whole length of all corresponding non-core regions of any one of the foregoing sequences.

Specific embodiments comprise a vaccine antigen variant sequence of SEQ ID NO:13, 31, 33, or 35, which comprises the detoxifying mutations as described herein, and which is particularly characterized by at least any one of 95%, 96%, 97%, 98%, 99%, or 99.5% sequence identity to the respective regions of any one of the foregoing sequences, in particular over the whole length of all corresponding regions of any one of the foregoing sequences.

Alternative combinations comprised in a vaccine antigen described herein, are complexes, wherein said molecules are adsorbed, adhered or otherwise immobilized or bound to a liposomal, nanoparticle or solid carrier, such as those suitable for use in formulating vaccine preparations.

Alternative combinations are mixtures of molecules, such as provided in a vaccine formulation at a predefined ratio or dose, for example, a mixture formulated on an adjuvant compound, such as an insoluble metal salt e.g., alum, aluminum hydroxide, or aluminum phosphate.

The present disclosure further provides for a pharmaceutical preparation comprising the antigen described herein, further comprising a pharmaceutically acceptable carrier, e.g., in an immunogenic formulation.

The invention further provides for a protective vaccine preparation comprising the antigen described herein, and a pharmaceutically acceptable carrier.

Specific embodiments of the vaccine preparation comprise an adjuvant, such as suitably used in human vaccine preparations.

Specific examples of adjuvants are selected from the group consisting of an insoluble metal salt, a glucopyranosyl Lipid A adjuvant, adjuvant systems which are stimulants of innate immunity, such as AS01, AS03, or AS04, MF59, a TCR stimulant such as a toll-like receptor agonist or CpG oligonucleotides, preferably alum, aluminum hydroxide, or aluminum phosphate.

Specifically, the vaccine can be administered to a subject, in particular a human subject, in an effective amount employing a prime-boost strategy.

Specifically, the effective amount of the vaccine antigen is ranging between 0.0001 and 1 mg per administration, preferably at least any one of 100, 200, 300, 400, 500, 600, 700, 800, 900 ng, or at least any one of 1, 2, 3, 4, 5, 6, 7, 8, 9, 10, 20, 30, 40, 50, 60, 70, 80, 90, or 100 µg, per dose.

Specifically, the vaccine preparation described herein is provided in a formulation for human use. Specific embodiments are provided in a formulation for parenteral such as intramuscular or subcutaneous, intranasal, mucosal, oral, microneedle skin, transdermal, sublingual, aerosolic or inhaled administration. Preferably, treatment comprises administration of the vaccine described herein by the respective route of administration.

The vaccine is specifically protective at a site of SEB exposure or toxicity, such as at one or more of the following sites: subcutaneous, mucosal gastrointestinal, skin or intramuscular sites.

According to a specific aspect, the vaccine preparation is a multivalent vaccine comprising said vaccine antigen in a combination with one or more Staphylococcal superantigen toxoid antigens, preferably selected from the group consisting of TSST-1, alpha-hemolysin, gamma-hemolysin, beta-hemolysin, staphylococcal exotoxins or enterotoxins, such as e.g., enterotoxin A (SEA), C (SEC), I (SEI), and K (SEK).

According to a specific aspect, Specifically, the detoxified SEB toxin or the vaccine antigen described herein is provided for medical, diagnostic or analytical use.

The present disclosure further provides for the medical use of the detoxified SEB toxin, the vaccine antigen or the vaccine preparation described herein, and in particular the use of such material in a method of producing a pharmaceutical preparation, such as a vaccine preparation, for treating a subject e.g., a human subject or patient, in particular for the prevention or therapy of specific disease conditions or diseases.

Specifically, the medical use involves an immunotherapy, such as an active immunotherapy. Specific immunotherapies provide for the treatment of a subject afflicted with, or at risk of contracting or suffering a disease or recurrence of a disease, by a method comprising inducing, enhancing, suppressing or otherwise modifying an immune response.

Specifically, the vaccine preparation is provided for use in the prevention or treatment of a Staphylococcal superantigen-expressing bacterial infection, and/or a disease condition directly or indirectly mediated by exposure to Staphylococcal superantigen toxins. Specifically, the disease condition is caused or triggered by a target Staphylococcus species.

Specifically, the vaccine preparation is provided for vaccinating a subject for prophylactic treatment to prevent infection with a Staphylococcus species, in particular vaccination and immunizing a subject in need thereof.

According to a specific aspect, the vaccine preparation may be used for preventing or treating any disease, disease condition or disorder in which inhibition, reduction of the growth of a Staphylococcus species would be beneficial.

According to a specific aspect, the vaccine preparation may be used for preventing or treating any disease, disease condition or disorder, which is associated with direct or indirect exposure to a Staphylococcal toxin, such as SEB. Such treatment may be indicated in a method of prevention or therapy of poisoning with such toxins, such as may arise from food poisoning or poisoning upon contact e.g., through mucosal or skin contact, or by inhalation of Staphylococcal toxins.

According to a specific aspect, the vaccine preparation may be used for preventing or treating a disease condition which is a sepsis condition induced by microbial mediators of Gram-positive bacterial, viral or fungal pathogens or toxins. Specifically, the sepsis condition is sepsis, toxic shock syndrome (TSS) or septic shock.

Microbial toxins, such as LPS or similar toxins of Gram-positive bacterial, viral or fungal pathogens may trigger severe sepsis conditions in a subject that has a predisposition of staphylococcal complications e.g., upon prior contact with Staphylococcal toxins and/or a Staphylococcus infection.

According to a specific aspect, the vaccine preparation may be used for immunizing a patient with the vaccine preparation, who is at risk of staphylococcal complications following a surgical intervention or invasive treatment of disease, preferably wherein the disease condition is a sepsis condition induced by microbial mediators of Gram-positive bacterial, viral or fungal pathogens or toxins, preferably wherein the sepsis condition is sepsis, toxic shock syndrome (TSS) or septic shock.. Immunizing such patient prior to such surgical intervention or invasive treatment would reduce the risk of staphylococcal complications.

The invention further provides for the use of the detoxified SEB toxin or the vaccine antigen as described herein, in a method of:
a) producing a vaccine preparation, or
b) producing antibodies specifically recognizing the SEB toxin.

The vaccine preparation may be manufactured by a method suitably used to prepare protein or subunit vaccines. Specifically, the vaccine preparation is produced by formulating the detoxified SEB toxin or the vaccine antigen in a vaccine formulation suitably used for the chosen route of administration.

The invention further provides for a method of producing the detoxified SEB toxin or the vaccine antigen as described herein, by expressing a nucleic acid sequence encoding the respective toxin or antigen in a recombinant host cell, and isolating and optionally purifying the respective toxin or antigen.

Specifically, the detoxified SEB toxin or the vaccine antigen as described herein, is provided as a recombinant protein such as produced by recombinant expression technologies.

The invention further provides for a nucleic acid molecule encoding the detoxified SEB toxin or the vaccine antigen as described herein. Such nucleic acid molecule may be codon-optimized, such that the risk of reverse mutations is reduced, or to improve expressing the sequence in a recombinant host organism, such as prokaryotic or eukaryotic or insect host cells, e.g., *E*. *coli,* yeast or mammalian expression systems.

Specific embodiments refer to a nucleic acid molecule which comprises or consists of any one of SEQ ID NO:2, 4, 6, 8, 10, 12, 14, 32, 34, or 36, or codon-optimized variants of any of the foregoing.

The present disclosure further provides for a recombinant expression construct for producing the detoxified SEB toxin or the vaccine antigen as described herein, in a recombinant host cell, comprising the nucleic acid molecule as described herein. Such expression construct comprises at least an expression cassette e.g., within a vector or plasmid, or be provided for chromosomal integration into the host cell genome.

According to a specific aspect, the present disclosure further provides for an expression system for producing the detoxified SEB toxin or the vaccine antigen as described herein, in an *ex vivo* cell culture, by a recombinant host cell comprising the nucleic acid molecule described herein.

The invention further provides for a recombinant host cell comprising the nucleic acid molecule described herein in an expression construct.

Suitable host cells may be selected from the group consisting of bacterial host cells, such as *E*. *coli,* but also from mammalian, insect, or yeast cells, e.g., HEK293 cells, CHO cells, NS0 cells, Sf9 cells, High Five cells, *Pichia pastoris, Saccharomyces cerevisiae,* among many others.

According to a specific aspect, the present disclosure further provides for a method of producing the detoxified SEB toxin or the vaccine antigen as described herein, wherein a recombinant host cell described herein is cultivated or maintained under conditions to produce said antigen.

### FIGURES

Figure 1 shows sequences referred to herein.
Figure 2. SEB toxin induces high T-cell proliferation in human peripheral blood mononuclear cells (PBMC) as detected by 3 H-thymidine incorporation in the range of 10mg to 1pg.
Figure 3. Proliferation of human PMNCs stimulated with SEB del 22-24: The mutant SEB del 22-24 induces highly reduced, but not absent, T-cell proliferation.
Figures 4-6 show improvements of SEB del 22-24 compared to SEB del L45R. The data are confirmed by

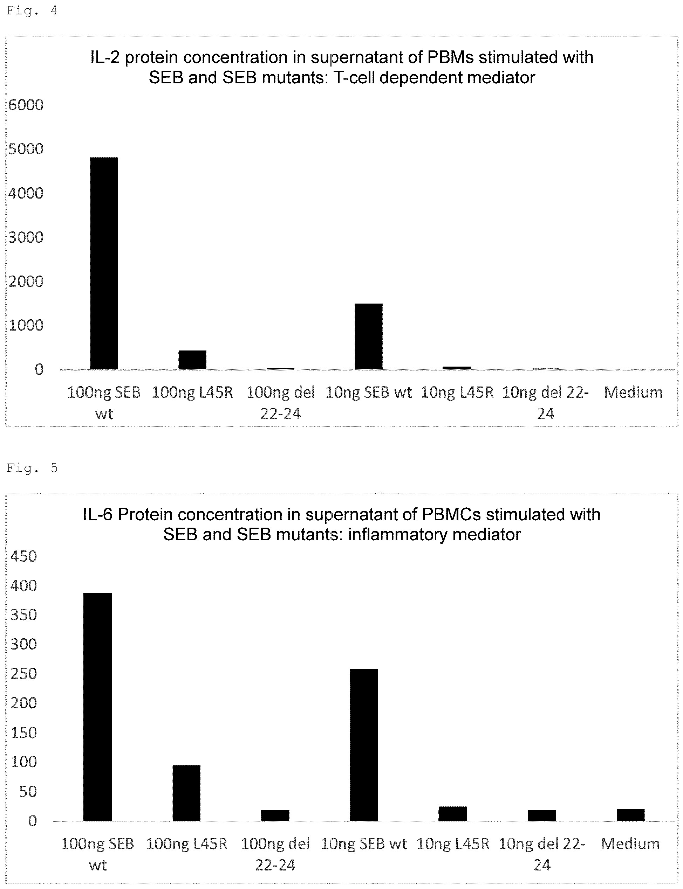

Figure 4. IL-2 protein concentration in supernatant of PBMs stimulated with SEB and SEB mutants, T-cell dependent mediator: SEB toxin causes dose-dependent high T-cell activation and in consequence, high IL-2 cytokine release. The double mutant variant L45R and deletion 22-24 do not activate T-cells, even in high concentrations, and II-2 cytokine release is not increased as compared to unstimulated cells.
Figure 5. IL-6 Protein concentration in supernatant of PBMCs stimulated with SEB and SEB mutants, inflammatory mediator: Well-known inflammatory response in humans in vivo; detection of and dose-dependent IL-6 release in human PBMC.

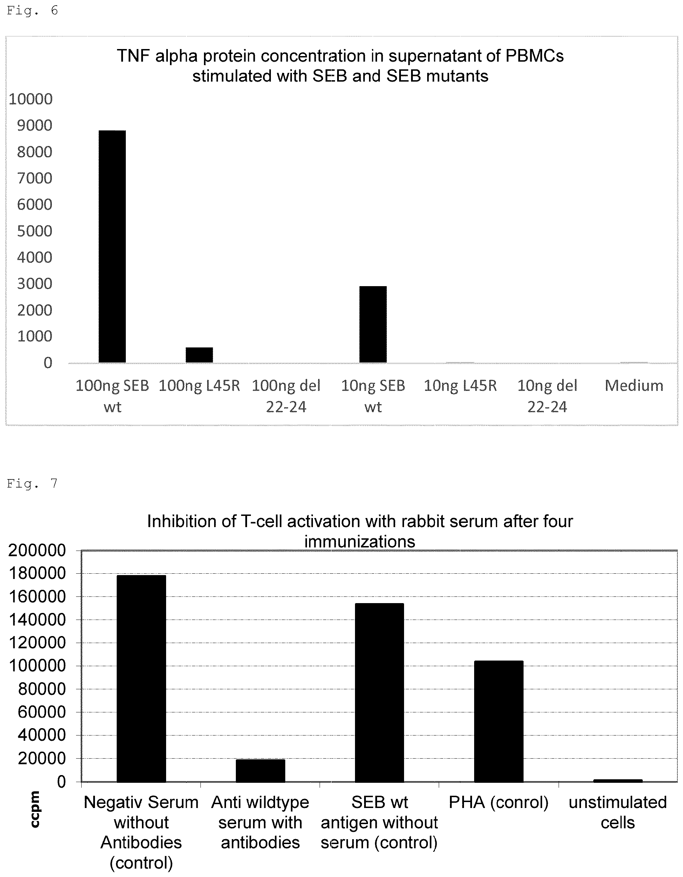

Figure 6. TNF alpha protein concentration in supernatant of PBMCs stimulated with SEB and SEB mutants. The effect is dose-dependent. Variant L45R and the deletion mutant 22-24 do not show TNF alpha release above the unstimulated control.
Figure 7. Inhibition of T-cell activation with rabbit serum after four immunizations.

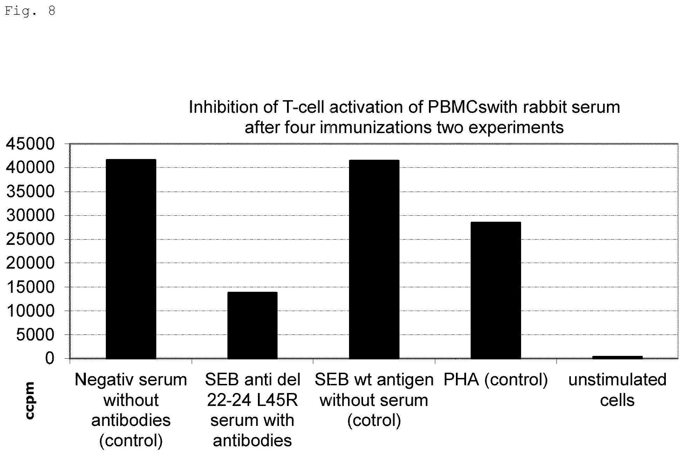

Figure 8. Inhibition of T-cell activation with rabbit serum after four immunizations: Antisera against SEB del 22-24 L45R, compared to antisera against SEB wild-type.

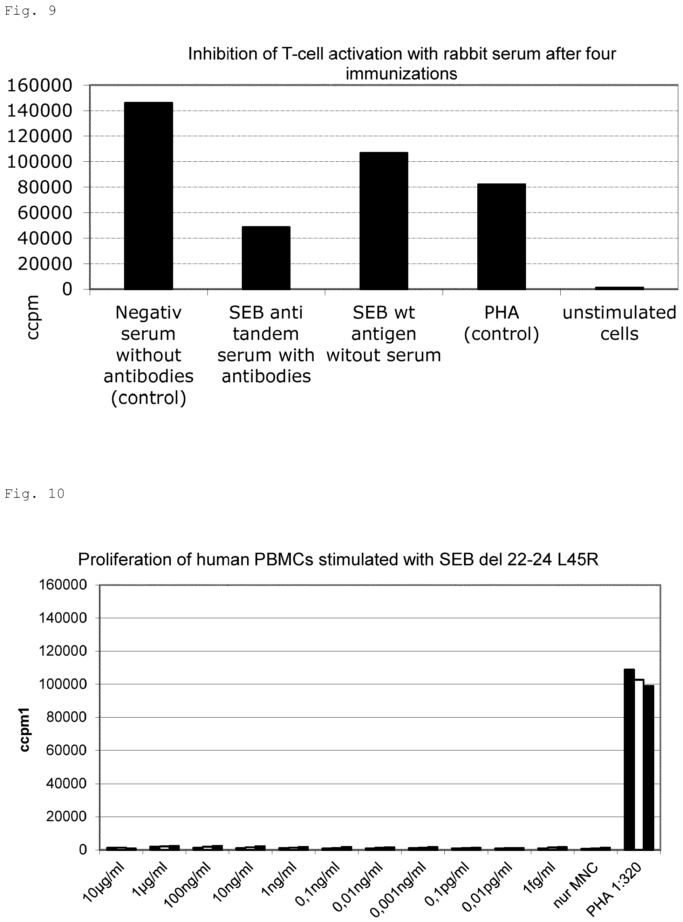

Figure 9. Inhibition of T-cell activation with rabbit serum after four immunizations: Antisera against a fusion (tandem) SEB del 22-24 L45R construct, compared to antisera against SEB wild-type.
Figure 10. Proliferation of human PBMCs stimulated with SEB del 22-24 L45R. T-cell proliferation in human PBMCs is highly reduced.

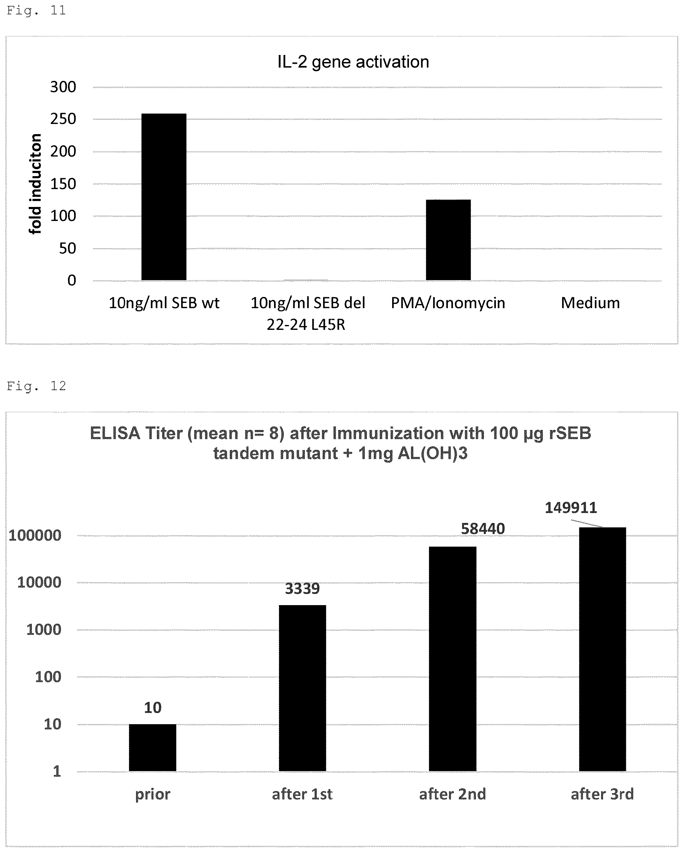

Figure 11. IL-2 gene activation after stimulation with SEB wildtype and SEB del 22-24 L45R. IL-2 gene activation is highly reduced with the double mutant.
Figure 12. ELISA Titer (mean n= 8) after Immunization with 100 µg a tandem SEB del 22-24 L45R construct + 1mg AL(OH)s.

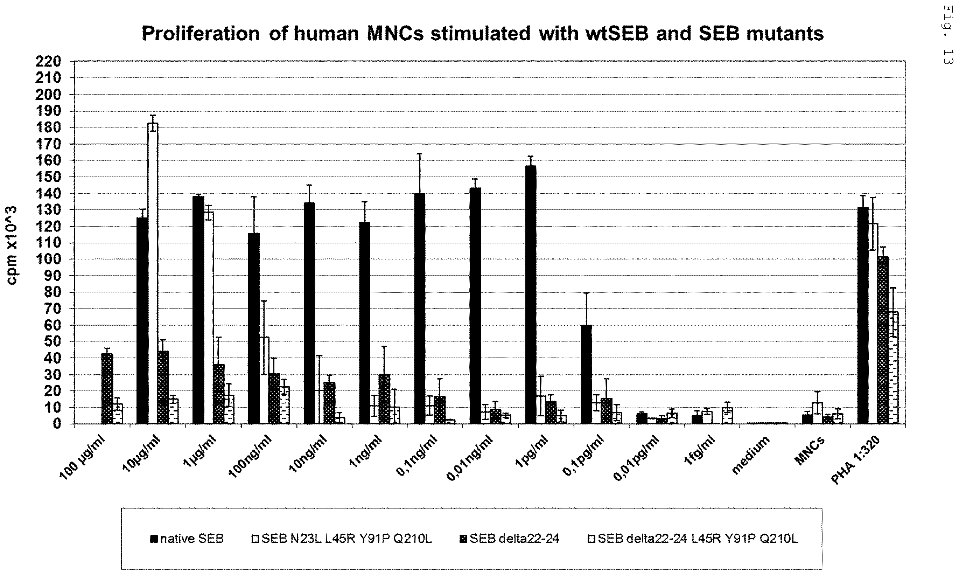

Figure 13: Proliferation of human MNCs, comparison of wtSEB, rSEB (del22-24), rSEB (N23L, L45R, Y91P, Q210L) and rSEB (del22-24, L45R, Y91P, Q210L).

### DETAILED DESCRIPTION OF THE INVENTION

Specific terms as used throughout the specification have the following meaning.

The terms "comprise", "contain", "have" and "include" as used herein can be used synonymously and shall be understood as an open definition, allowing further members or parts or elements. "Consisting" is considered as a closest definition without further elements of the consisting definition feature. Thus "comprising" is broader and contains the "consisting" definition.

The term "antigen" as used herein shall in particular refer to any antigenic determinant, which can be possibly recognized by a binding site of an antibody. Specifically, preferred antigens are those molecules or structures, which have already been proven to be or are capable of being immunologically or therapeutically relevant, especially those, for which a clinical efficacy has been tested. The term as used herein shall in particular comprise molecules or structures selected from antigens comprising immunoaccessible and immunorelevant epitopes, in particular conserved antigens found in one or more species or serotype. Immunoaccessible viral epitopes are typically presented by or comprised in antigens expressed on the outer surface of a virion or on the surface of an infected cell.

Selected epitopes and peptides as described herein may trigger an immune response *in vivo,* so to induce neutralizing antibodies against the antigen and target bacteria, respectively. This provides for the effective protection upon active immunization with the antigen. Polypeptide antigens are preferred antigens due to their inherent ability to elicit both cellular and humoral immune responses.

Protective antigens referred to herein are understood to induce a protective immune response.

The term "protective immune response" or "protective immunity" is herein understood as follows: Protective immunity is a condition of a subject conferred by the immune response generated by immunization, such that the subject's response is sufficient to survive or neutralize a challenging dose of the respective antigen or pathogen, even a dose that would be otherwise toxic or lethal in a non-immunized subject, or which response inhibits an undesired or uncontrolled inflammation and/or activation of immune cells. Staphylococcal toxins like SEB may cause activation of antigen-presenting cells and/or T cells and uncontrolled inflammatory reactions which can damage organs e.g., gastrointestinal, respiratory, urogenital, mucosal or brain damage, or can even be lethal. The subject vaccines described herein are specifically designed to confer protective immunity to prevent or reduce such inflammatory reactions. Protective immunity can be determined by *in vitro* or *in vivo* assays determining inhibition of undesired stimulation of antigen-presenting cells and/or T cell activation. *In vivo* assays preferably employ suitable animal models, preferably in at least two different species, e.g., mouse and rabbit models.

The antigens described herein are understood as protein antigens specifically comprising a linear, unbranched amino acid sequence, which is naturally-occurring but modified to introduce certain detoxifying mutations, which may be further modified, e.g., by mutation or chemical derivatization, such as by phosphorylation, methylation, acetylation, amidation, formation of pyrrolidone carboxylic acid, isomerization, hydroxylation, sulfation, flavin-binding, cysteine oxidation and nitrosylation.

The detoxified toxins and vaccine antigens used herein are specifically designed to trigger an immune response in a subject, and particularly include one or more antigenic determinants, which can be possibly recognized by a binding site of an antibody or are able to bind to the peptide groove of HLA class I or class II molecules or other antigen presenting molecules such as CD1 and as such may serve as stimulant for specific T-cells. The vaccine antigens specifically include one or more immunologically relevant epitopes, which are herein understood as structures that are recognized by the subject's immune system and/or respective antibodies. Epitopes can e.g., be B-cell epitopes or T-cell epitopes, such as CD4+ T-cell epitopes or CD8+ T-cell epitopes.

Neutralizing activity of an immune response or respective neutralizing antibodies can be tested in cell-based assays and *in vivo.* Neutralizing antibodies can be determined e.g., by enumerating bacterial titers in the presence of antibodies and/or by detecting a cytopathic effect in cell-based infection assays.

Protective immune responses can be measured using *in vivo* models of Staphylococcus infection.

The term "expression" is understood in the following way. Nucleic acid molecules containing a desired coding sequence of an expression product such as e.g., a detoxified SEB toxin or a vaccine antigen as described herein, and control sequences such as e.g. a promoter in operable linkage, may be used for expression purposes. Hosts transformed or transfected with these sequences are capable of producing the encoded proteins. In order to effect transformation, the expression system may be included in a vector; however, the relevant DNA may also be integrated into the host cell chromosome. Specifically, the term refers to a host cell and compatible vector under suitable conditions, e.g., for the expression of a protein coded for by foreign DNA carried by the vector and introduced to the host cell.

Coding DNA is a DNA sequence that encodes a particular amino acid sequence for a particular polypeptide or protein. Promoter DNA is a DNA sequence which initiates, regulates, or otherwise mediates or controls the expression of the coding DNA. Promoter DNA and coding DNA may be from the same gene or from different genes, and may be from the same or different organisms. Recombinant cloning vectors will often include one or more replication systems for cloning or expression, one or more markers for selection in the host, e.g., antibiotic resistance, and one or more expression cassettes.

"Vectors" used herein are defined as DNA sequences that are required for the transcription of cloned recombinant nucleotide sequences, i.e., of recombinant genes and the translation of their mRNA in a suitable host organism.

An "expression cassette" refers to a DNA coding sequence or segment of DNA that code for an expression product that can be inserted into a vector at defined restriction sites. The cassette restriction sites are designed to ensure insertion of the cassette in the proper reading frame. Generally, foreign DNA is inserted at one or more restriction sites of the vector DNA, and then is carried by the vector into a host cell along with the transmissible vector DNA. A segment or sequence of DNA having inserted or added DNA, such as an expression vector, can also be called a "DNA construct".

Expression vectors comprise the expression cassette and additionally usually comprise an origin for autonomous replication in the host cells or a genome integration site, one or more selectable markers (e.g., an amino acid synthesis gene or a gene conferring resistance to antibiotics such as zeocin, kanamycin, G418 or hygromycin), a number of restriction enzyme cleavage sites, a suitable promoter sequence and a transcription terminator, which components are operably linked together. The term "vector" as used herein includes autonomously replicating nucleotide sequences as well as genome integrating nucleotide sequences. A common type of vector is a "plasmid", which generally is a self-contained molecule of double-stranded DNA that can readily accept additional (foreign) DNA and which can readily be introduced into a suitable host cell. A plasmid vector often contains coding DNA and promoter DNA and has one or more restriction sites suitable for inserting foreign DNA. Specifically, the term "vector" or "plasmid" refers to a vehicle by which a DNA or RNA sequence (e.g., a foreign gene) can be introduced into a host cell, so as to transform the host and promote expression (e.g., transcription and translation) of the introduced sequence.

The term "host cell" as used herein shall refer to primary subject cells transformed to produce a particular recombinant protein, and any progeny thereof. It should be understood that not all progeny is exactly identical to the parental cell (due to deliberate or inadvertent mutations or differences in environment), however, such altered progeny are included in these terms, so long as the progeny retain the same functionality as that of the originally transformed cell. The term "host cell line" refers to a cell line of host cells as used for expressing a recombinant gene to produce recombinant proteins. The term "cell line" as used herein refers to an established clone of a particular cell type that has acquired the ability to proliferate over a prolonged period of time. Such host cell or host cell line may be maintained in cell culture and/or cultivated to produce a recombinant polypeptide.

The detoxified toxins and vaccine antigens used herein are specifically provided as isolated proteins. The term "isolated" or "isolation" as used herein with respect to a protein shall refer to such compound that has been sufficiently separated from the environment with which it would naturally be associated, so as to exist in "purified" or "substantially pure" form. Yet, "isolated" does not necessarily mean the exclusion of artificial or synthetic fusions or mixtures with other compounds or materials, or the exclusion of impurities that do not interfere with the fundamental activity, and that may be present, for example, due to incomplete purification. Isolated compounds can be further formulated to produce preparations thereof, and still for practical purposes be isolated - for example, a mixture of proteins or the respective fusion proteins described herein can be mixed with pharmaceutically acceptable carriers, including those which are suitable for analytic, diagnostic, prophylactic or therapeutic applications, or excipients when used in diagnosis, medical treatment, or for analytical purposes.

The term "purified" as used herein shall refer to a preparation comprising at least 50% (w/w total protein), preferably at least 60%, 70%, 80%, 90% or 95% of a compound (e.g., a chimeric antigen described herein). A highly purified product is essentially free from contaminating proteins, and preferably has a purity of at least 70%, more preferred at least 80%, or at least 90%, or even at least 95%, up to 100%. Purity is measured by methods appropriate for the compound (e.g., chromatographic methods, polyacrylamide gel electrophoresis, HPLC analysis, and the like). An isolated, purified protein described herein may be obtained as a recombinant product obtained by purifying from a host cell culture expressing the product in the cell culture supernatants, to reduce or remove host cell impurities or from cellular debris.

As isolation and purification methods for obtaining a purified protein, methods utilizing difference in solubility, such as salting out and solvent precipitation, methods utilizing difference in molecular weight, such as ultrafiltration and gel electrophoresis, methods utilizing difference in electric charge, such as ion-exchange chromatography, methods utilizing specific affinity, such as affinity chromatography, methods utilizing difference in hydrophobicity, such as reverse phase high performance liquid chromatography, and methods utilizing difference in isoelectric point, such as isoelectric focusing may be used. Standard methods can be used such as cell (debris) separation and wash by Microfiltration or Tangential Flow Filter (TFF) or centrifugation, protein purification by precipitation or heat treatment, protein activation by enzymatic digest, protein purification by chromatography, such as ion exchange (IEX), hydrophobic interaction chromatography (HIC), Affinity chromatography, size exclusion (SEC) or HPLC chromatography, protein precipitation of concentration and washing by ultrafiltration steps. An isolated and purified protein can be identified by conventional methods such as Western blot, HPLC, activity assay, or ELISA.

The term "nucleic acid molecule" used herein refers to either DNA (including e.g. cDNA) or RNA (including e.g. mRNA) molecules comprising a polynucleotide sequence. The molecule may be a single or double-stranded polymer of deoxyribonucleotide or ribonucleotide bases read from the 5' to the 3' end. The term includes coding sequences, such as genes, artificial polynucleotides such as comprised in an expression construct expressing the respective polypeptide sequence.

Nucleic acid molecules encoding the detoxified SEB molecules or vaccine antigens described herein are specifically provided by mutagenesis (mutation) of naturally-occurring SEB toxin sequences. Mutagenesis to delete specific amino acids involves the deletion of one or more nucleotides. Mutagenesis to substitute one specific amino acid may involve substitution of at least 1 or 2 nucleotides, wherein the codon comprising said substituted nucleotides encodes the substituted amino acid. Where nucleotides are substituted, it is preferred to exchange more than one nucleotide within a codon, to reduce the risk of undesired spontaneous reverse mutation. Codonoptimization of a nucleotide sequence and molecule described herein may involve increasing the number of substituted nucleotides within a codon, such as to improve the stability of the mutants, and/or avoiding reverse mutation by only one spontaneous nucleotide exchange.

A DNA or RNA molecule can be used which comprises a nucleotide sequence that is degenerate to any of the sequences or a combination of degenerate sequences, or which comprises a codon-optimized sequence to improve expression in a host. For example, an *E*. *coli* codon optimized sequence can be used. Specific RNA molecules can be used to provide a respective RNA-vaccine.

Expression systems, genetic constructs or modifications described herein may employ tools, methods and techniques known in the art, such as described by J. Sambrook et al., Molecular Cloning: A Laboratory Manual (3rd edition), Cold Spring Harbor Laboratory, Cold Spring Harbor Laboratory Press, New York (2001). Expression vectors may include but are not limited to cloning vectors, modified cloning vectors and specifically designed plasmids. Preferred expression vectors described herein are expression vectors suitable for expressing of a recombinant gene in a bacterial or eukaryotic host cell and are selected depending on the host organism. Appropriate expression vectors typically comprise regulatory sequences suitable for expressing DNA encoding a recombinant protein in a eukaryotic host cell. Examples of regulatory sequences include promoter, operators, enhancers, ribosomal binding sites, and sequences that control transcription and translation initiation and termination. The regulatory sequences are typically operably linked to the DNA sequence to be expressed.

The term "naturally-occurring" as used herein shall refer to those elements, sequences or products which are found in nature, such as expressed by or found in native, wild-type organism. Specifically, the detoxified toxins or vaccine antigens described herein comprise mutations which are not naturally-occurring, i.e., which are artificial ("man-made") but artificial mutants e.g., produced employing a mutagenesis technology.

A recombinant nucleic acid may be one that has a sequence that is not naturally-occurring or that has a sequence that is made by an artificial combination of two otherwise separated segments of sequence. This artificial combination is often accomplished by chemical synthesis or, more commonly, by the artificial manipulation of isolated segments of nucleic acids, e.g., by genetic engineering techniques well-known in the art. For example, a nucleic acid can be chemically synthesized using naturally-occurring nucleotides or variously modified nucleotides designed to increase the biological stability of the molecules or to increase the physical stability of the duplex formed upon hybridization.

Mutants of a protein, such as comprising detoxifying mutations as described herein, may be provided e.g., by introducing a certain number of point mutations into a parent aa sequence. Specifically, a mutagenesis method is used to introduce one or more point mutations.

The term "detoxifying mutations" as used herein shall refer to those one or more point mutation(s) in either or both of the T cell receptor binding region or the immunoglobulin superfamily binding region of a naturally-occurring (wild-type) SEB toxin. Such regions are herein also referred to as "core regions". Any other regions of the SEB toxin are herein also referred to as "non-core regions". Detoxifying mutations reduce, diminish or abolish the protein's toxicity, thereby improving the safety upon administration to a subject.

A point mutation as described herein is typically at least one of a deletion, insertion, and/or substitution of one or more nucleotides within a nucleotide sequence to achieve the deletion, insertion, and/or substitution of one (only a single one) amino acid at a certain, defined position within the amino acid sequence encoded by said nucleotide sequence. Therefore, the term "point mutation" as used herein shall refer to a mutation of a nucleotide sequence or an amino acid sequence. Specifically, preferred point mutations are substitutions, in particular non-conservative or conservative ones. Conservative substitutions are those that take place within a family of amino acids that are related in their side chains and chemical properties. Examples of such families are amino acids with basic side chains, with acidic side chains, with non-polar aliphatic side chains, with non-polar aromatic side chains, with uncharged polar side chains, with small side chains, with large side chains etc. In this regard, amino acids refer to twenty naturally occurring amino acids encoded by sixty-four triplet codons. These 20 amino acids can be split into those that have neutral charges, positive charges, and negative charges. Preferred point mutations refer to the exchange of amino acids of the different polarity and/or charge.

Specific mutagenesis methods provide for point mutations of one or more nucleotides in a sequence, in some embodiments tandem point mutations, such as to change at least 2, 3, 4, or 5, or even more contiguous nucleotides within a nucleotide sequence of a parent molecule.

The term "mutagenesis" as used herein shall refer to a method of preparing or providing mutants of a nucleotide sequence and the respective protein encoded by said nucleotide sequence, e.g., through insertion, deletion and/or substitution of one or more nucleotides, so to obtain variants thereof with at least one change in the coding region. Mutagenesis may be through site directed, random, or semi-random. A mutagenesis method can encompass methods of engineering the nucleic acid or *de novo* synthesizing a nucleotide sequence using the respective parent sequence information as a template. For instance, a library of nucleotide sequences may be prepared by mutagenesis of a selected parent nucleotide sequence. A library of variants may be produced and a suitable mutant protein sequence be selected according to a specifically desired genotype or phenotype.

According to a specific embodiment, any of the detoxified toxins and vaccine antigens used herein may be produced as a recombinant protein, such as produced by recombinant DNA technology. As used herein, the term "recombinant" refers to a molecule or construct that does not naturally occur in a host cell. In some embodiments, recombinant nucleic acid molecules contain two or more naturally-occurring sequences that are linked together in a way that does not occur naturally. A recombinant protein refers to a protein that is encoded and/or expressed by a recombinant nucleic acid. In some embodiments, "recombinant cells" express genes that are not found in identical form within the native (i.e., non-recombinant) form of the cell and/or express native genes that are otherwise abnormally over-expressed, under-expressed, and/or not expressed at all due to deliberate human intervention. Recombinant cells contain at least one recombinant polynucleotide, polypeptide or protein. "Recombination", "recombining", and generating a "recombined" nucleic acid generally encompass the assembly of at least two nucleic acid fragments.

The term "recombinant" as used herein specifically means "being prepared by or the result of genetic engineering" i.e., by human intervention. A recombinant nucleotide sequence may be engineered by introducing one or more point mutations in a parent nucleotide sequence, and may be expressed in a recombinant host cell that comprises an expression cassette including such recombinant nucleotide sequence. The polypeptide expressed by such expression cassette and host cell, respectively, is also referred to as being "recombinant". For the purpose described herein conventional molecular biology, microbiology, and recombinant DNA techniques within the skill of the art may be employed. Specific embodiments described herein refer to the production of a chimeric antigen, and the recombinant means for such production, including a nucleic acid encoding the amino acid sequence, an expression cassette, a vector or plasmid comprising the nucleic acid encoding the amino acid sequence to be expressed, and a host cell comprising any such means. Suitable standard recombinant DNA techniques are known in the art and described *inter alia* in Sambrook et al., "Molecular Cloning: A Laboratory Manual" (1989), 2nd Edition (Cold Spring Harbor Laboratory press).

Herein the term "subject" is understood to comprise human or mammalian subjects, including livestock animals, companion animals, and laboratory animals, in particular human beings, which are either patients suffering from a specific disease condition or healthy subjects. The term "patient" includes human and other mammalian subjects that receive either prophylactic or therapeutic treatment. The term "patient" as used herein always includes healthy subjects.

In particular the treatment and medical use described herein applies to a subject in need of prophylaxis or therapy of a disease condition associated with a Staphylococcus infection and/or contamination. Specifically, the treatment may be by interfering with the pathogenesis of a disease condition where a Staphylococcus species is a causal agent of the condition. The subject may be a subject at risk of such disease condition or suffering from disease.

Non-limiting examples of some common disease conditions or disorders caused by *Staphylococcus aureus* bacteria or toxins comprise burns, cellulitis, skin necrosis, eyelid infections, food poisoning, joint infections, pneumonia, skin infections, surgical wound infection, scalded skin syndrome and toxic shock syndrome. Some of the disease conditions may relate to the direct, indirect or secondary effect of toxins on cell function and cell damage or lysis.

According to a specific aspect, the Staphylococcus species targeted by the vaccine as described herein are selected from human pathogenic Staphylococcus species, or those Staphylococcus species that cause an infection in humans. In embodiments, the Staphylococcus species comprises *Staphylococcus aureus, Staphylococcus epidermidis, Staphylococcus lugdunensis,* and/or *Staphylococcus saprophyticus,* in particular including antibiotic resistant species such as methicillin resistant Staphylococcus aureus, and including mutant pathogenics which may evolve during an infection, or mutants that are artificially evolved.

In specific embodiment, a treatment method is provided to prevent an infection with a Staphylococcus species in a subject, and/or to ameliorate a disease condition that may arise in a subject at risk of staphylococcal complications. The vaccine preparation described herein may be specifically used to treat diseases or disorders associated with Staphylococcus infection, especially infection with antibiotic resistant species, including a skin infection, cellulitis, pneumonia, meningitis, urinary tract infection, toxic shock syndrome, endocarditis, pericarditis, osteomyelitis, bacteremia, or sepsis. The vaccine preparation described herein may also be used to prevent Staphylococcus infections and/or staphylococcal complications that may arise during or following a surgical procedure, such as surgery that involves implantation of a pacemaker, artificial heart valve, a joint implant, or a prosthetic. In certain embodiments, the prosthetic may be a prosthetic limb, such as an arm or a leg. In certain embodiments, the prosthetic may be a hip replacement. In certain embodiments, the prosthetic may be a cosmetic prosthetic, such as, but not limited to an ocular prosthetic, silicone hands, fingers, breasts, feet, toes, or a facial implant. In some embodiments, a subject is treated who has undergone, or who is planning on undergoing a surgical procedure, wherein the subject is at risk of acquiring a Staphylococcus infection and/or staphylococcal complications.

Staphylococcal complications are specifically those associated with *Staphylococcus aureus* bacteremia or intoxication. Although most staphylococcus infections are not serious, S. *aureus* can cause serious infections such as bloodstream infections, pneumonia, or bone and joint infections, including e.g., acute complications (such as sepsis, toxic shock syndrome (TSS), septic shock, adult respiratory distress syndrome, disseminated intravascular coagulation, etc.), or chronic (including relapsing) diseases (such as osteomyelitis, endocarditis, infections of indwelling devices and wound infections). Patients at risk of complications of *Staphylococcus aureus* Infection include those receiving hemodialysis, injection drug users, patients with diabetes, and patients with nasal carriage of S. *aureus,* in particular methicillin-resistant S. *aureus,* and/or those with preexisting cardiac conditions or other comorbidities.

The term "at risk of" a certain disease conditions, refers to a subject that potentially develops such a disease condition, e.g., by a certain predisposition, exposure to Staphylococcus bacteria or toxins, or that already suffers from a respective disease condition at various stages, particularly associated with other causative disease conditions or else conditions or complications following as a consequence of viral infection.

The term "treatment" as used herein shall always refer to treating subjects for prophylactic (i.e., to prevent infection and/or disease status) or therapeutic (i.e. to treat diseases regardless of their pathogenesis) purposes.

The term "prophylaxis" as used herein refers to preventive measures which is intended to encompass prevention of the onset of pathogenesis or prophylactic measures to reduce the risk of pathogenesis.

The term "therapy" as used herein with respect to treating subjects refers to medical management of a subject with the intent to cure, ameliorate, stabilize, reduce the incidence or prevent a disease, pathological condition, or disorder, which individually or together are understood as "disease condition".

The vaccine described herein specifically comprises the detoxified SEB toxin or the vaccine antigen in an effective amount, which is herein specifically understood as "immunologically effective amount". By "immunologically effective amount", it is meant that the administration of that amount to a subject, either in a single dose or as part of a series of doses, is effective on the basis of the therapeutic or prophylactic treatment objectives. A "prophylactically effective amount" refers to an amount effective, at dosages and for periods of time necessary, to achieve the desired prophylactic result, such as preventing a target Staphylococcus bacterial infection or a staphylococcal complication, or inhibiting diseases directly or indirectly caused by Staphylococcus bacteria or toxins. This amount will vary depending upon the health and physical condition of the subject to be treated, age, the capacity of the subject's immune system to synthesize antibodies, the type and degree of immune response desired, the formulation of the vaccine, and other conditions.

An effective amount or dosage may range from 0.0001 to 2 mg, e.g., between 0.001 and 2 mg, of the vaccine antigen administered to the subject in need thereof, e.g., an adult human subject. For example, the effective dosage of the vaccine antigen is capable of eliciting an immune response in a subject of effective levels of antibody titer to bind and neutralize one or more target Staphylococcus bacteria or toxins, e.g., 1-3 months after immunization. The effectiveness can be assayed by the respective antibody titers in samples of blood taken from the subject.

In some embodiments, an effective amount is one that has been correlated with beneficial effect when administered as part of a particular dosing regimen, e.g., a single administration or a series of administrations such as in a "boosting" regimen. For treatment, the vaccine described herein may be administered at once, or may be divided into the individual components and/or a number of smaller doses to be administered at intervals of time. Typically, upon priming a subject by a first injection of a vaccine described herein, one or more booster injections may be performed over a period of time by the same or different administration routes. Where multiple injections are used, subsequent injections may be made, e.g., within 1 to 52 weeks of the previous injection.

The vaccine described herein may comprise the detoxified SEB toxin or the vaccine antigen in an immunogenic formulation. Specific embodiments comprise one or more adjuvants and/or pharmaceutically acceptable excipients or carriers.

Pharmaceutical carriers suitable for facilitating certain means of administration are well known in the art. Specific embodiments refer to immunogenic formulations, which comprise a pharmaceutically acceptable carrier and/or adjuvant, which trigger a humoral (B-cell, antibody) or cytotoxic (T-cell) immune response. Adjuvants may specifically be used to enhance the effectiveness of the vaccine. Adjuvants may be added directly to the vaccine compositions or can be administered separately, either concurrently with or shortly before or after administration of the vaccine antigen.

The term "adjuvant" as used herein specifically refers to a compound that when administered in conjunction with an antigen augments and/or redirects the immune response to the antigen, but when administered alone does not generate an immune response to the antigen. Adjuvants can augment an immune response by several mechanisms including lymphocyte recruitment, stimulation of B- and/or T-cells, and stimulation of macrophages.

An "effective amount" of an adjuvant can be used in a vaccine described herein, which is specifically understood to be an amount which enhances an immunological response to the immunogen such that, for example, lower or fewer doses of the immunogenic composition are required to generate a specific immune response and a respective effect of preventing or combating bacterial infection, intoxication, or disease.

The term "pharmaceutically acceptable carrier" denotes one or more non-toxic material effectiveness of biological activity does not interfere with active ingredients, including but not limited to buffer solution, preservative, compatible carrier and optionally other additives or encapsulating substances. The term "carrier" denotes a natural or synthetic organic or inorganic ingredient and active ingredient combined with the carrier to facilitate application. Pharmaceutically acceptable carriers generally include any and all suitable solvents, dispersion media, coatings, antibacterial and antifungal agents, isotonic and absorption delaying agents, and the like that are physiologically compatible with an antibody or related composition or combination provided by the invention. Specific examples of pharmaceutically acceptable carriers include sterile water, saline, phosphate buffered saline, dextrose, glycerol, ethanol, polyethylene glycol, and the like, as well as combinations of any thereof. Additional pharmaceutically acceptable carriers are known in the art and described in, e.g., Remington: The Science and Practice of Pharmacy, 22nd revised edition (Allen Jr, LV, ed., Pharmaceutical Press, 2012). Liquid formulations can be solutions, emulsions or suspensions and can include excipients such as suspending agents, solubilizers, surfactants, preservatives, and chelating agents. Exemplary carriers are carrier proteins such as selected from tetanus toxoid, diphtheria toxoid and CRM₁₉₇, or liposomes or cationic peptides; exemplary adjuvants are alum, aluminum phosphate or aluminum hydroxide, MF59 or CpG oligonucleotides. Further adjuvants include (in)complete Freund's adjuvant, *B. pertussis* or its toxin, or 1031.

The preferred preparation is in a ready-to-use, storage stable form, with a shelflife of at least one or two years. The invention also provides a delivery device, e.g. a syringe, pre-filled with the vaccine as described herein.

The vaccine described herein can be administered by conventional routes known the vaccine field, in such as to a mucosal (e.g., ocular, intranasal, pulmonary, oral, gastric, intestinal, rectal, vaginal, or urinary tract) surface, via a parenteral (e.g., subcutaneous, intradermal, intramuscular, intravenous, or intraperitoneal) route, or topical administration (e.g., via a patch). The choice of administration route depends upon a number of parameters, such as the adjuvant associated with the polypeptide. If a mucosal adjuvant is used, the intranasal, oral or inhaled route is preferred. If a lipid formulation or an aluminum compound is used, the parenteral route is preferred with the sub-cutaneous or intramuscular route being most preferred.

The injectable preparations may include dosage forms for subcutaneous, intracutaneous and intramuscular injections, etc. These injectable preparations may be prepared by methods publicly known. For example, the injectable preparations may be prepared, e.g., by dissolving, suspending or emulsifying the protein in a sterile aqueous medium or an oily medium conventionally used for injections. As aqueous medium for injections, there are, for example, physiological saline, an isotonic solution containing glucose and other auxiliary agents, etc., which may be used in combination with an appropriate solubilizing agent such as an alcohol (e.g., ethanol), a polyalcohol (e.g., propylene glycol, polyethylene glycol), a nonionic surfactant e.g., polysorbate 80, HCO-50 (polyoxyethylene (50 mol) adduct of hydrogenated castor oil), etc. As oily medium, there are employed, e.g., sesame oil, soybean oil, etc., which may be used in combination with a solubilizing agent such as benzyl benzoate, benzyl alcohol, etc. The injection thus prepared is preferably filled in an appropriate ampoule.

The foregoing description will be more fully understood with reference to the following examples. Such examples are, however, merely representative of methods of practicing one or more embodiments of the present invention and should not be read as limiting the scope of invention.

### EXAMPLES

### Example 1: Producing SEB mutants, vaccine antigen and preparation

The SEB toxin gene was isolated from *S*. *aureus* (ATCC 14458, toxin sequence SEQ ID NO:1; nucleotide sequence SEQ ID NO:2), and is genetically modified to lose its toxic properties. Specific amino acids that determine the binding sites for the immunoglobulin superfamily and the T-cell receptor have been changed on the DNA level so that SEB protein cannot bind to either receptor, and its toxic characteristic is lost.

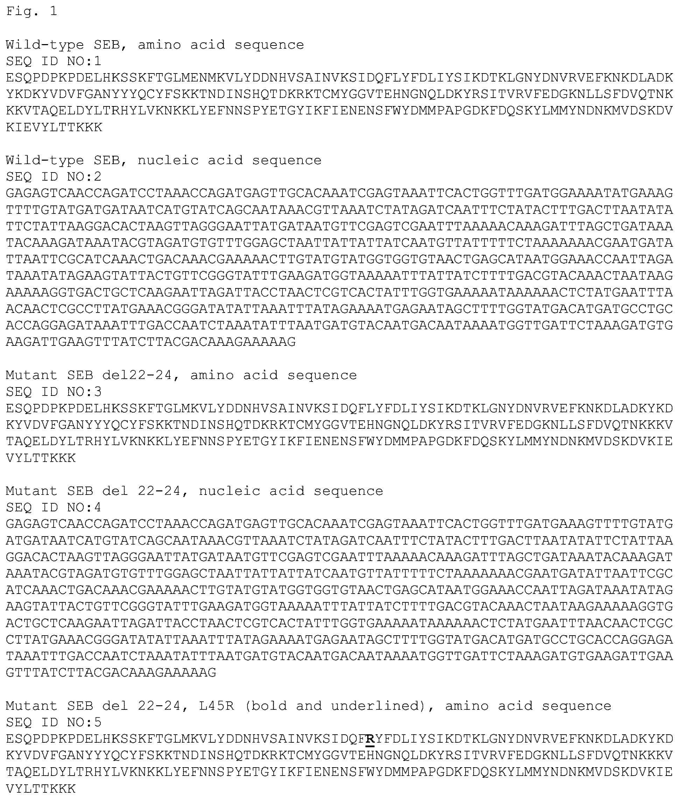

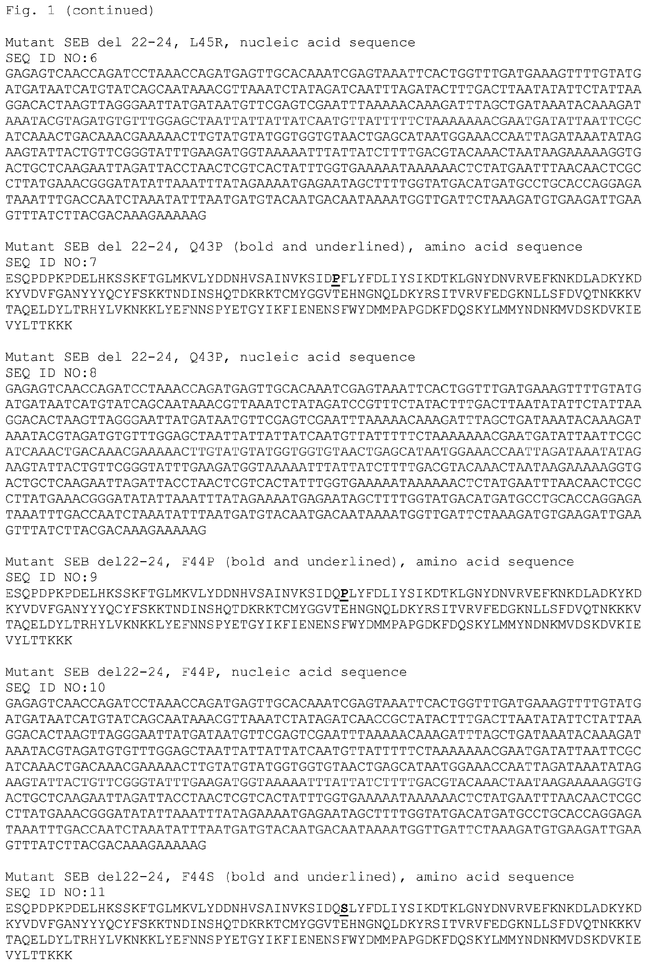

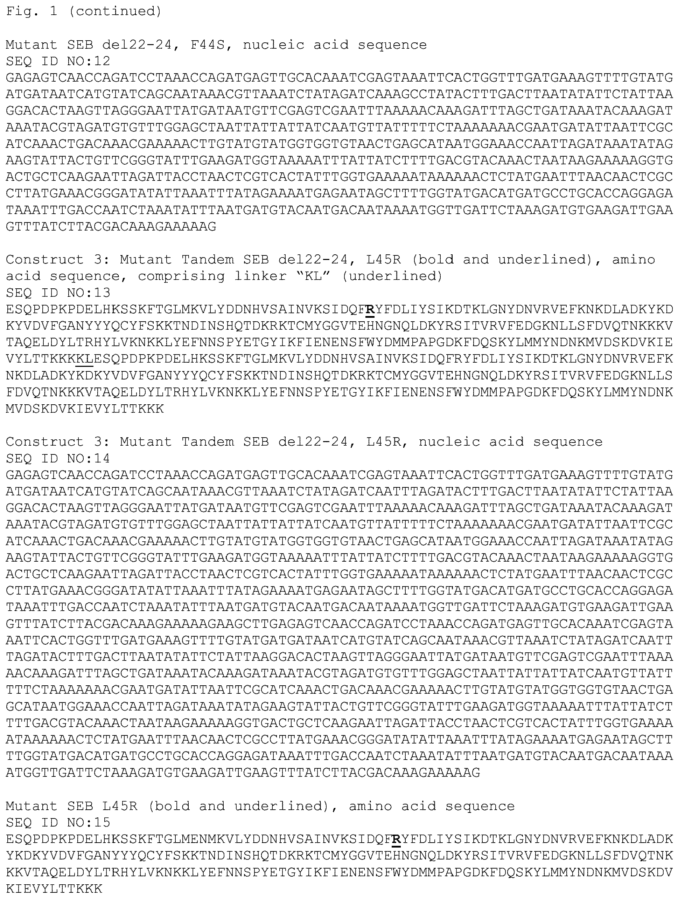

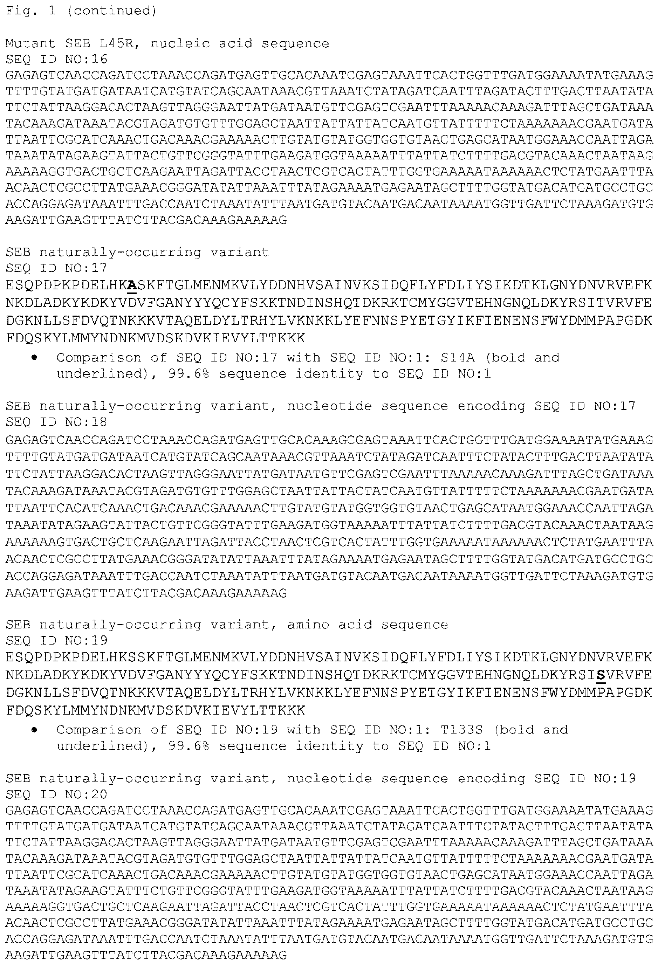

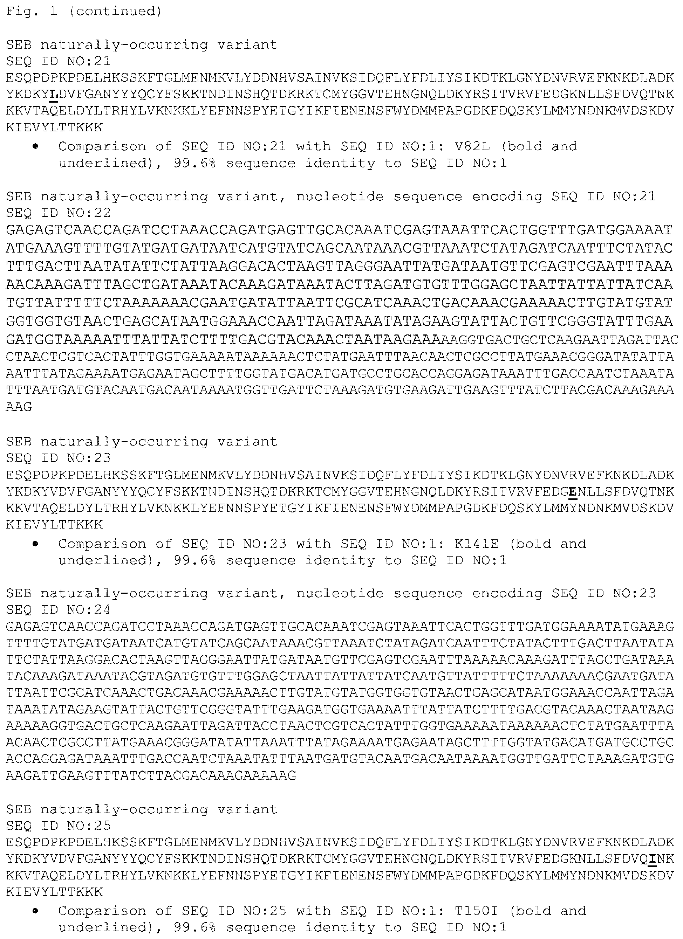

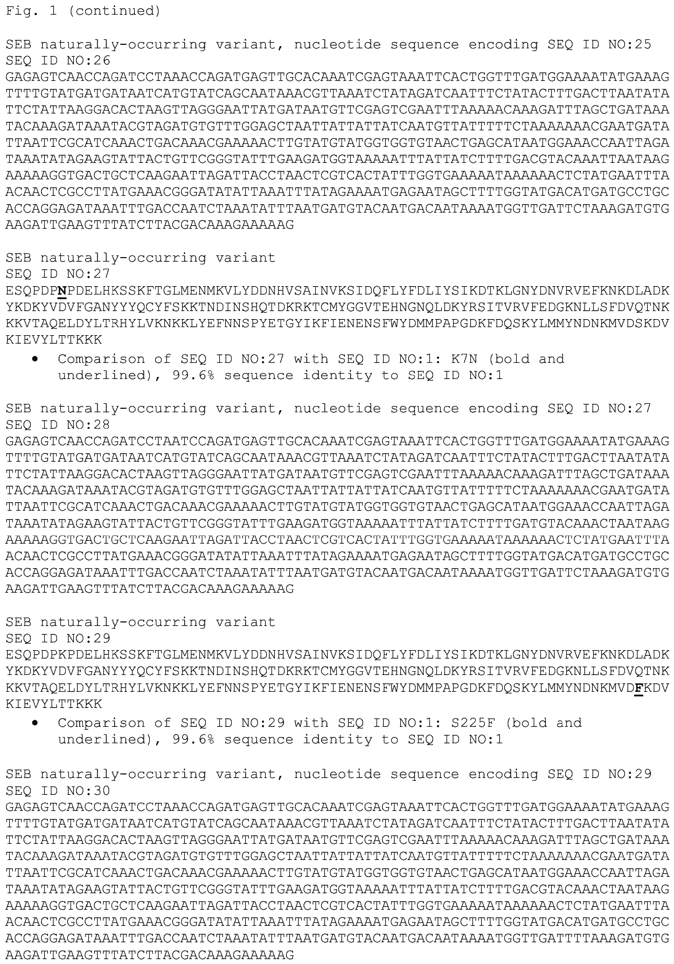

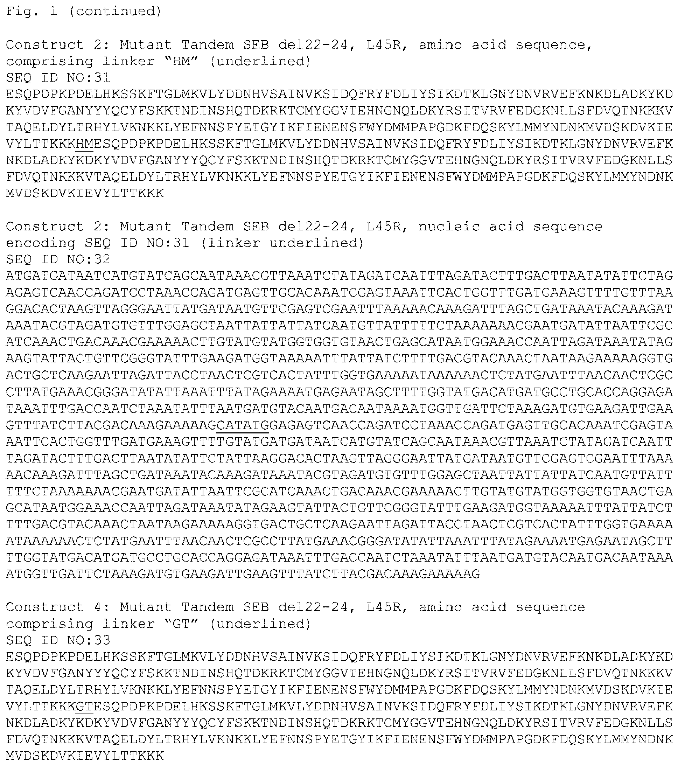

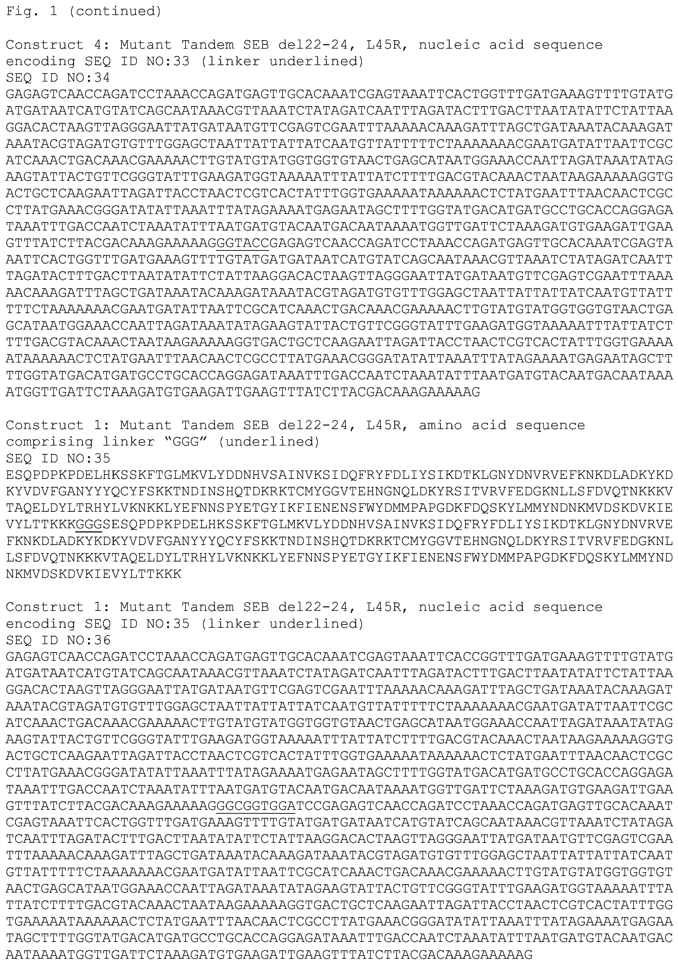

Mutagenesis of the TCR binding site is performed by deletion of amino acids 22 to 24. The immunoglobulin superfamily binding region is mutated by exchanging amino acid 45 from leucine to arginine. The SEB single and double mutants were compared with the wild-type. The respective sequences are provided in Figure 1.

SEB wild-type and its mutants were expressed in *E.coli* BL21-AI (invitrogen) using a pET-9d plasmid (Novagen) and tested for immunogenicity and lack of toxicity in comparison to wild type SEB. Recombinant proteins were purified by chromatography and formulated with Al(OH)₃ as a vaccine.

A tandem repeat construct resulting in a coding sequence of 1428 nucleotides was produced to express a recombinant detoxified SEB tandem variant which is used as vaccine antigen.

### Mutation of SEB

### a) Deletion of aa22-24 in SEQ ID NO:1

Deletion of 9 nucleotides was performed using a reverse primer binding upstream of the desired deletion and a forward primer binding downstream of the deletion. In two different PCR reactions DNA of the wild type was amplified from the 5'-end of the DNA to the deletion and from the deletion to the 3'-end. Both fragments were ligated and in a final PCR the deletion mutant was amplified.

The resulting nucleotide sequence is identified as SEQ ID NO:4, encoding the protein sequence SEQ ID NO:3.

### b) L45R Mutation in SEQ ID NO:1

The point mutation L45R was created using an internal reverse primer where codon CTA was mutated to AGA. In two different PCR reactions DNA of the wild type was amplified from the 5'-end of the DNA to the point mutation and past the mutation to the 3'-end. Both fragments were ligated and in a final PCR the deletion mutant was amplified.

The resulting nucleotide sequence is identified as SEQ ID NO:15, encoding the protein sequence SEQ ID NO:16.

### c) Double mutants and tandem mutants: SEB del 22-24+L45R (also referred to as SEB del22-24 L45R)

The following double and tandem mutants have been prepared accordingly.
SEB del 22-24, L45R
SEB del 22-24, Q43P
SEB del22-24, F44P
SEB del22-24, F44S
Tandem SEB del22-24, L45R using various linker sequences

In tandem double mutants, two double mutant molecules are fused using a linker sequence. An exemplary construct encodes SEQ ID NO:13 including a linker of two amino acids (Lys-Leu) between the tandem repeats.

The following tandem repeat constructs of SEB double mutant (del 22-24 and L45R) were produced as described above using different linker sequences, each referred to as rSEB Variant Vaccine. Construct 1 comprises a GGG linker, Constructs 2-4 each comprises a linker consisting of two amino acids, selected from His, Met, Lys, Leu, Gly, and Thr. The sequences are provided in Figure 1.

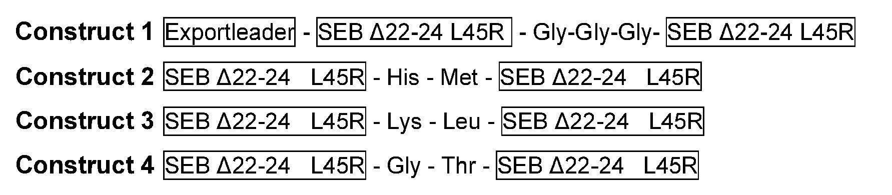

### Example 2: Safety

Toxicity was determined by the following standard assays:
a) T-cell proliferation in human peripheral blood mononuclear cells (PBMC) as detected by 3H-thymidine incorporation;
b) T-cell activation and in consequence, dose-dependent IL-2 cytokine release; and
c) inflammatory response in human PBMCs; detection of dose-dependent IL-6 release, and TNF alpha release.

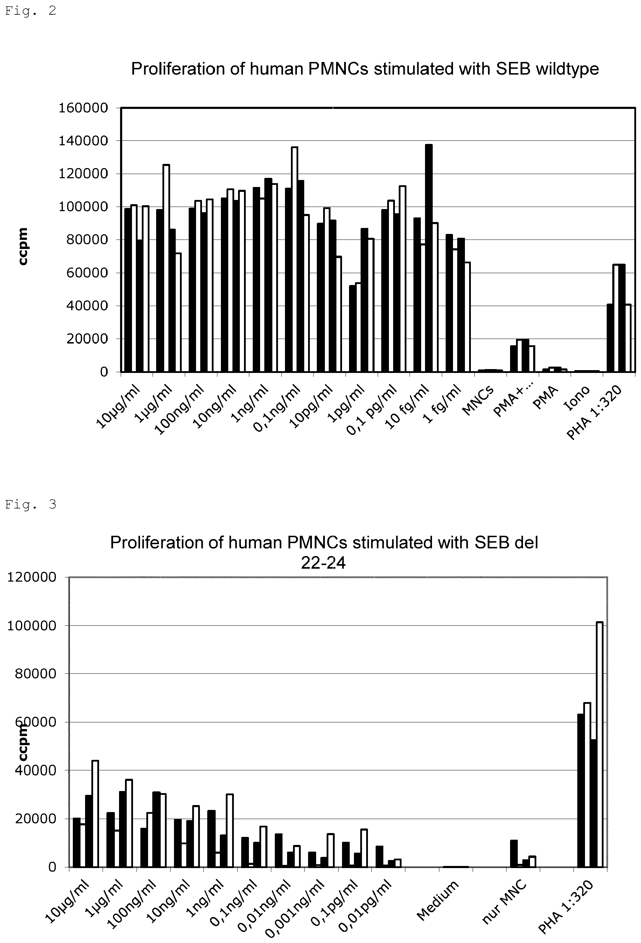

The results are provided in Figures 2-6, and in Figures 10-11. The mutant SEB del 22-24 and SEB del 22-24 L45R highly reduce T cell proliferation in human peripheral blood mononuclear cells (PBMC) as compared to SEB wild-type toxin. Both, the double mutant variant SEB del 22-24 L45R and SEB del 22-24 do not activate T-cells, even at high concentrations, and II-2 cytokine release is not increased as compared to unstimulated cells. IL-6 release in human PBMCs and TNF alpha release was dose dependent, and greatly reduced by the SEB del 22-24 mutation. Figures 4-6 show an improved toxicity profile of SEB del 22-24 compared to SEB del L45R.

SEB del 22-24 L45R showed no T-cell activation in human PBMCs up to 10 µg/ml and no IL-2 gene activation. No signs of toxicity were detected when given as a bolus injection to rabbits.

Each of the fusion Constructs 1-4 was non-toxic as proven by *in vitro* and *in vivo* toxicity tests: Neither of the four constructs induced T-cell activation of human PBMCs up to 10 µg/ml. All tandem constructs neither induced IL-2 gene activation and IL-2 release, nor induced proinflammatory cytokine (IFN gamma, IL-6, TNF alpha) gene activation or secretion. No signs of toxicity were detected when given as a bolus injection to rabbits or as a high dose into mice.

### Example 3: Immunogenicity and protection in mice and rabbits

Rabbits were immunized four times with 10 µg, 30 µg or 100 µg rSEB Variant Constructs 1 to 4, using 1mg AL(OH)s as adjuvant. Sera were analyzed by ELISA and neutralizing antibodies were determined by a neutralization assay (inhibition of T-cell activation with rabbit antisera).

Protective immunity was induced in a rabbit model of immunization. Antisera were produced and tested for protectivity, using standard assays.

Results are shown in Figures 7-9.

Binding titers and neutralization titers were achieved with SEB del 22-24 L45R. Immunogenicity of SEB del 22-24 L45R was even improved by all of the tested tandem repeat Constructs 1-4 (rSEB Variant Constructs). Construct 3 indicated the best results.

Figure 12 shows the ELISA Titer (mean n= 8) after Immunization with 100 µg of a tandem SEB del 22-24 L45R construct + 1mg AL(OH)s.

Protection was achieved with SEB del 22-24 L45R and rSEB Variant Constructs, after three or four immunizations challenged with 30 µg and 100 µg SEB toxin.

### Protective immunity in mice and rabbits

Mice were immunized two times or three times with various doses (1 µg to 30 µg) of a rSEB Variant construct. Two weeks after the last immunization, the mice were challenged with 20 µg SEB toxin and after four hours with 40 µg LPS. All challenged mice survived (see Tables 3 and 4).

**Table 3. Two times immunized mice challenged with SEB toxin and LPS**

| Immunization | Challenge | no of dead mice dead/challenqed mice |
|---|---|---|
| 2 x 1µg rSEB Variant | 20µg SEB + 10µg LPS | 0/8 |
| 2 x 3µg rSEB Variant | 20µg SEB + 10µg LPS | 0/10 |
| 2 x 10µg rSEB Variant | 20µg SEB + 10µg LPS | 0/10 |
| 2 x 30µg rSEB Variant | 20µg SEB + 10µg LPS | 0/10 |

**Table 4. Three times immunized mice challenged with SEB toxin and LPS**

| Immunization | Challenge | no of dead mice dead/challenged mice |
|---|---|---|
| 3 x 1µg rSEB Variant | 20µg SEB + 10µg LPS | 0/10 |
| 3 x 3µg rSEB Variant | 20µg SEB + 10µg LPS | 0/10 |
| 3 x 10µg rSEB Variant | 20µg SEB + 10µg LPS | 0/10 |
| 3 x 30µg rSEB Variant | 20µg SEB + 10µg LPS | 0/10 |

Protection was demonstrated with the rSEB Variant Constructs after two and three immunizations in mice and after three immunizations in rabbits challenged with SEB toxin and LPS.Rabbits were immunized three times with 30 µg rSEB Variant Construct 3 + 1mg AL(OH)s. Ten days after the last immunization, the rabbits were challenged with 30 µg SEB toxin and after four hours with 10 µg LPS. All three rabbits survived the challenge.

### Example 4: improved tolerability of rSEB comprising a deletion of aa22-24

Toxicity: Comparing proliferation of human PMNCs stimulated with any one of:
wtSEB (native SEB),
SEB N23L L45R Y91P Q210L
SEB del 22-24
SEB del22-24 L45R Y91P Q210L.

Reference is made to Figure 13.

Mutant SEB del22-24 L45R Y91P Q210L shows decreased toxicity compared to the quadruple point mutant SEB N23L L45R Y91P Q210L without any deletion of amino acids 22-24. The deletion mutant del22-24 shows comparable toxicity to SEB del22-24 L45R Y91P Q210L, and lower toxicity as the quadruple point mutant SEB N23L L45R Y91P Q210L.

Discussion: The SEB mutant comprising a deletion within amino acids 21-25, in this example aa22-24, improved tolerability as compared to the substitution of N23L. Such effect is independent on the additional point mutations in the MHC Class II receptor binding region, at positions 45, 91 and 210.

## Claims

1. **A detoxified Staphylococcal Exotoxin B (SEB) toxin** that is mutated to comprise three point mutations at amino acid positions 21 to 25 in the SEB toxin sequence SEQ ID NO:1, wherein said three point mutations comprise a deletion of aa21-23 or aa22-24, or a deletion at corresponding amino acid positions in any other naturally-occurring SEB toxin sequence that has at least 95% sequence identity to SEQ ID NO:1.

2. The detoxified SEB toxin of claim 1, comprising or consisting of SEQ ID NO:3 which differs from SEQ ID NO:1 in the deletion of aa22-24, or a detoxified SEB variant sequence thereof comprising said deletion of aa22-24, and at least 95% sequence identity to SEQ ID NO:3.

3. The detoxified SEB toxin of claim 1 or 2, comprising one or more further point mutations comprising at least one amino acid substitution at a position selected from the group consisting L45, Q43, or F44, preferably L45R, Q43P, F44P, or F44S.

4. The detoxified SEB toxin of claim 3, wherein the detoxified SEB toxin comprises or consists of any one of SEQ ID NO:5, 7, 9, or 11, or a detoxified SEB variant sequence of any one of the foregoing comprising said three point mutations at amino acid positions 21 to 25 and at least 95% sequence identity to of any one of the foregoing.

5. **A protective vaccine antigen** comprising at least one molecule of the detoxified SEB toxin of any one of claims 1 to 4, preferably in combination with a Staphylococcal vaccine antigen that is identical to or different from said detoxified SEB toxin molecule, optionally wherein the combination is provided as a fusion, mixture, or a complex comprising said molecule and antigen bound to each other and/or bound to a carrier, thereby forming the complex.

6. The vaccine antigen of claim 5, wherein said at least one detoxified SEB toxin molecule is fused to another detoxified SEB toxin molecule, optionally comprising a linker sequence.

7. The vaccine antigen of claim 6, comprising or consisting of any one of SEQ ID NO:13, 31, 33, or 35, or a vaccine antigen variant sequence of any one of the foregoing comprising said three point mutations at amino acid positions 21 to 25 and at least 95% sequence identity to of any one of the foregoing.

8. **A protective vaccine preparation** comprising the antigen of any one of claims 5 to 7, and a pharmaceutically acceptable carrier.

9. The vaccine preparation of claim 8, comprising an adjuvant.

10. The vaccine preparation of claim 8, wherein the adjuvant is selected from the group consisting of an insoluble metal salt, a glucopyranosyl Lipid A adjuvant, adjuvant systems which are stimulants of innate immunity, such as AS01, AS03, or AS04, MF59, a TCR stimulant such as a toll-like receptor agonist or CpG oligonucleotides, preferably alum, aluminum hydroxide, or aluminum phosphate.

11. The vaccine preparation of any one of claims 8 to 10, which is provided in a formulation for intramuscular, subcutaneous, intranasal, mucosal, oral, microneedle skin, transdermal, sublingual, aerosolic or inhaled administration.

12. The vaccine preparation of any one of claims 8 to 11, which is a multivalent vaccine comprising said vaccine antigen in a combination with one or more Staphylococcal superantigen toxoid antigens, preferably selected from the group consisting of TSST-1, alpha-hemolysin, gamma-hemolysin, beta-hemolysin, and staphylococcal exotoxins or enterotoxins, such as enterotoxin A (SEA), C (SEC), I (SEI), and K (SEK).

13. The vaccine preparation of any one of claims 8 to 12, for use in the prevention or treatment of a Staphylococcal superantigen-expressing bacterial infection, and/or a disease condition directly or indirectly mediated by exposure to Staphylococcal superantigen toxins.

14. The vaccine preparation for use according to claim 13, wherein a patient is immunized with the vaccine preparation, who is at risk of staphylococcal complications following a surgical intervention or invasive treatment of disease, preferably wherein the disease condition is a sepsis condition induced by microbial mediators of Gram-positive bacterial, viral or fungal pathogens or toxins, preferably wherein the sepsis condition is sepsis, toxic shock syndrome (TSS) or septic shock.

15. Use of the detoxified SEB toxin of any one of claims 1 to 4, or the vaccine antigen of any one of claims 5 to 7, in an in vitro method of
a) producing a vaccine preparation, or
b) producing antibodies specifically recognizing the SEB toxin.

16. A method of producing the detoxified SEB toxin of any one of claims 1 to 4, or the vaccine antigen of any one of claims 5 to 7, by expressing a nucleic acid sequence encoding the respective toxin or antigen in a recombinant host cell, and isolating and optionally purifying the respective toxin or antigen.

17. A nucleic acid molecule encoding the detoxified SEB toxin of any one of claims 1 to 4, or the vaccine antigen of any one of claims 5 to 7, preferably comprising or consisting of any one of SEQ ID NO:2, 4, 6, 8, 10, 12, 14, 32, 34, or 36, or codon-optimized variants of any of the foregoing.

18. A recombinant host cell comprising the nucleic acid molecule of claim 17 in an expression construct.

## Patentansprüche

1. **Enttoxifiziertes Staphylokokken-Exotoxin-B- bzw. SEB-Toxin,** das mutiert ist, so dass es drei Punktmutationen an den Aminosäure-Positionen 21 bis 25 in der SEB-Toxin-Sequenz SEQ ID NO:1 umfasst, wobei die drei Punktmutationen eine Deletion von aa21-23 oder aa22-24, oder eine Deletion an entsprechenden Aminosäure-Positionen in einer beliebigen anderen natürlich vorkommenden SEB-Toxin-Sequenz umfassen, die mindestens 95 % Sequenzidentität mit SEQ ID NO:1 aufweist.

2. Enttoxifiziertes SEB-Toxin nach Anspruch 1, das eine SEQ ID NO:3 umfasst oder aus dieser besteht, die sich von SEQ ID NO:1 in der Deletion von aa22-24 unterscheidet, oder eine enttoxifizierte Sequenzvariante davon, welche die Deletion von aa22-24 umfasst, und mindestens 95 % Sequenzidentität mit SEQ ID NO:3.

3. Enttoxifiziertes SEB-Toxin nach Anspruch 1 oder 2, umfassend eine oder mehrere weitere Punktmutationen, die mindestens eine Aminosäure-Substitution an einer Position umfassen, die ausgewählt ist aus der Gruppe bestehend aus L45, Q43 oder F44, vorzugsweise L45R, Q43P, F44P oder F44S.

4. Enttoxifiziertes SEB-Toxin nach Anspruch 3, wobei das enttoxifizierte SEB-Toxin eine beliebige von SEQ ID NO:5, 7, 9 oder 11 umfasst oder daraus besteht, oder eine enttoxifizierte SEB-Sequenzvariante aus einer beliebigen der vorstehend genannten, welche die drei Punktmutationen an den Aminosäure-Positionen 21 bis 25 und mindestens 95 % Sequenzidentität mit einer beliebigen der vorstehend genannten umfasst.

5. **Schutzimpfstoff-Antigen,** umfassend mindestens ein Molekül des enttoxifizierten SEB-Toxins nach einem der Ansprüche 1 bis 4, vorzugsweise in Kombination mit einem Staphylokokken-Impfstoff-Antigen, das identisch mit oder unterschiedlich von dem enttoxifizierten SEB-Toxin-Molekül ist, wobei die Kombination optional als eine Fusion, Mischung oder ein Komplex bereitgestellt wird, die/der das Molekül und das Antigen aneinandergebunden und/oder an einen Träger gebunden umfasst, wodurch der Komplex gebildet wird.

6. Impfstoff-Antigen nach Anspruch 5, wobei das mindestens eine enttoxifizierte SEB-Toxin-Molekül mit einem weiteren enttoxifizierten SEB-Toxin-Molekül fusioniert ist, optional umfassend eine Linkersequenz.

7. Impfstoff-Antigen nach Anspruch 6, das beliebig eine von SEQ ID NO:13, 31, 33 oder 35 umfasst oder daraus besteht, oder eine Impfstoff-Antigen-Sequenzvariante aus einer beliebigen der vorstehend genannten, welche die drei Punktmutationen an den Aminosäure-Positionen 21 bis 25 und mindestens 95 % Sequenzidentität mit einer beliebigen der vorstehend genannten umfasst.

8. **Schutzimpfstoff-Zubereitung,** umfassend das Antigen nach einem der Ansprüche 5 bis 7, und einen pharmazeutisch annehmbaren Träger.

9. Impfstoff-Zubereitung nach Anspruch 8, umfassend ein Adjuvans.

10. Impfstoff-Zubereitung nach Anspruch 8, wobei das Adjuvans ausgewählt ist aus der Gruppe bestehend aus einem unlöslichen Metallsalz, einem Glucopyranosyl-Lipid-A-Adjuvans, Adjuvanzien-Systemen, welche die angeborene Immunität stimulieren, wie etwa ASO1, ASO3 oder ASO4, MF59, einem TCR-Stimulator wie etwa einem Toll-ähnlicher-Rezeptor-Agonist oder CpG-Oligonukleotiden, vorzugsweise Alaun, Aluminiumhydroxid oder Aluminiumphosphat.

11. Impfstoff-Zubereitung nach einem der Ansprüche 8 bis 10, die in einer Formulierung für die intramuskuläre, subkutane, intranasale, mucosale, orale, Mikronadel-Haut-, transdermale, sublinguale, aerosolische oder inhalierte Verabreichung bereitgestellt wird.

12. Impfstoff-Zubereitung nach einem der Ansprüche 8 bis 11, die einen mehrwertigen Impfstoff darstellt, der das Impfstoff-Antigen in einer Kombination mit einem oder mehreren Staphylokokken-Superantigen-Toxoid-Antigenen umfasst, vorzugsweise ausgewählt aus der Gruppe bestehend aus TSST-1, Alpha-Hämolysin, Gamma-Hämolysin, Beta-Hämolysin, und Staphylokokken-Exotoxinen oder - Enterotoxinen, wie etwa Enterotoxin A (SEA), C (SEC), I (SEI), und K (SEK).

13. Impfstoff-Zubereitung nach einem der Ansprüche 8 bis 12, zur Verwendung bei der Vorbeugung oder Behandlung einer staphylokokken-superantigen-exprimierenden bakteriellen Infektion und/oder eines Erkrankungszustands, der direkt oder indirekt durch Exposition gegenüber Staphylokokken-Superantigen-Toxinen vermittelt ist.

14. Impfstoff-Zubereitung zur Verwendung nach Anspruch 13, wobei ein Patient mit der Impfstoff-Zubereitung immunisiert wird, für welchen das Risiko von staphylokokken-bedingten Komplikationen infolge eines chirurgischen Eingriffs oder einer invasiven Behandlung einer Erkrankung besteht, wobei der Erkrankungszustand vorzugsweise ein Sepsis-Zustand ist, der durch mikrobielle Mediatoren von grampositiven bakteriellen, viralen oder fungalen Pathogenen oder Toxinen induziert wird, wobei der Sepsis-Zustand vorzugsweise Sepsis, toxisches Schocksyndrom (TSS) oder septischer Schock ist.

15. Verwendung des enttoxifizierten SEB-Toxins nach einem der Ansprüche 1 bis 4, oder des Impfstoff-Antigens nach einem der Ansprüche 5 bis 7, in einem Invitro-Verfahren zur
a) Herstellung einer Impfstoff-Zubereitung, oder
b) Herstellung von Antikörpern, die spezifisch das SEB-Toxin erkennen.

16. Verfahren zur Herstellung des enttoxifizierten SEB-Toxins nach einem der Ansprüche 1 bis 4, oder des Impfstoff-Antigens nach einem der Ansprüche 5 bis 7, durch Exprimieren einer Nukleinsäuresequenz, die für das jeweilige Toxin oder Antigen kodiert, in einer rekombinanten Wirtszelle, und Isolieren und gegebenenfalls Aufreinigen des jeweiligen Toxins oder Antigens.

17. Nukleinsäuremolekül, das für das enttoxifizierte SEB-Toxin nach einem der Ansprüche 1 bis 4, oder das Impfstoff-Antigen nach einem der Ansprüche 5 bis 7 kodiert, vorzugsweise umfassend oder bestehend aus einer beliebigen von SEQ ID NO:2, 4, 6, 8, 10, 12, 14, 32, 34 oder 36, oder codon-optimierten Varianten einer beliebigen der vorstehend genannten.

18. Rekombinante Wirtszelle, umfassend das Nukleinsäuremolekül nach Anspruch 17 in einem Expressionskonstrukt.

## Revendications

1. **Toxine exotoxine staphylococcique B (SEB) détoxifiée** qui est mutée pour comprendre trois mutations ponctuelles au niveau des positions d'acides aminés 21 à 25 dans la séquence de toxine SEB SEQ ID N° : 1, dans laquelle lesdites trois mutations ponctuelles comprennent une délétion des aa21 à 23 ou des aa22 à 24, ou une délétion au niveau des positions d'acides aminés correspondantes dans toute autre séquence de toxine SEB d'origine naturelle qui présente une identité de séquence d'au moins 95 % avec SEQ ID N° : 1.

2. Toxine SEB détoxifiée selon la revendication 1, comprenant ou constituée de SEQ ID N° : 3 qui diffère de SEQ ID N° : 1 dans la délétion des aa22 à 24, ou une séquence de variant de SEB détoxifiée de celle-ci comprenant ladite délétion des aa22 à 24, et une identité de séquence d'au moins 95 % avec SEQ ID N° : 3.

3. Toxine SEB détoxifiée selon la revendication 1 ou 2, comprenant une ou plusieurs mutations ponctuelles supplémentaires comprenant au moins une substitution d'acide aminé au niveau d'une position choisie dans le groupe constitué par L45, Q43 ou F44, de préférence L45R, Q43P, F44P ou F44S.

4. Toxine SEB détoxifiée selon la revendication 3, la toxine SEB détoxifiée comprenant ou étant constituée de l'une quelconque des SEQ ID N° : 5, 7, 9 ou 11, ou d'une séquence de variant de SEB détoxifiée de l'une quelconque des séquences précédentes comprenant lesdites trois mutations ponctuelles au niveau des positions d'acides aminés 21 à 25 et une identité de séquence d'au moins 95 % avec l'une quelconque des séquences précédentes.

5. **Antigène vaccinal protecteur** comprenant au moins une molécule de la toxine SEB détoxifiée selon l'une quelconque des revendications 1 à 4, de préférence en combinaison avec un antigène vaccinal staphylococcique qui est identique ou différent de ladite molécule de toxine SEB détoxifiée, éventuellement la combinaison étant fournie sous la forme d'une fusion, d'un mélange ou d'un complexe comprenant ladite molécule et un antigène liés l'un à l'autre et/ou liés à un vecteur, ce qui permet de former le complexe.

6. Antigène vaccinal selon la revendication 5, dans lequel ladite au moins une molécule de toxine SEB détoxifiée est fusionnée à une autre molécule de toxine SEB détoxifiée, comprenant éventuellement une séquence de liaison.

7. Antigène vaccinal selon la revendication 6, comprenant ou constitué de l'une quelconque des SEQ ID N° : 13, 31, 33 ou 35, ou d'une séquence de variant d'antigène vaccinal de l'une quelconque des séquences précédentes comprenant lesdites trois mutations ponctuelles au niveau des positions d'acides aminés 21 à 25 et une identité de séquence d'au moins 95 % avec l'une quelconque des séquences précédentes.

8. **Préparation vaccinale protectrice** comprenant l'antigène selon l'une quelconque des revendications 5 à 7, et un vecteur pharmaceutiquement acceptable.

9. Préparation vaccinale selon la revendication 8, comprenant un adjuvant.

10. Préparation vaccinale selon la revendication 8, dans laquelle l'adjuvant est choisi dans le groupe constitué par un sel métallique insoluble, un adjuvant glucopyranosyl lipide A, les systèmes d'adjuvants qui sont des stimulants de l'immunité innée, tels que AS01, AS03 ou AS04, MF59, un stimulant de TCR tel qu'un agoniste de récepteur de type toll ou les oligonucléotides de CpG, de préférence l'alun, l'hydroxyde d'aluminium ou le phosphate d'aluminium.

11. Préparation vaccinale selon l'une quelconque des revendications 8 à 10, qui est fournie dans une formulation pour une administration intramusculaire, souscutanée, intranasale, mucosale, orale, cutanée par micro-aiguille, transdermique, sublinguale, par aérosol ou par inhalation.

12. Préparation vaccinale selon l'une quelconque des revendications 8 à 11, qui est un vaccin multivalent comprenant ledit antigène vaccinal en combinaison avec un ou plusieurs antigènes d'anatoxines superantigéniques staphylococciques, de préférence choisis dans le groupe constitué par TSST-1, l'alpha-hémolysine, la gamma-hémolysine, la bêta-hémolysine et les exotoxines ou entérotoxines staphylococciques, telles que les entérotoxines A (SEA), C (SEC), I (SEI) et K (SEK).

13. Préparation vaccinale selon l'une quelconque des revendications 8 à 12, destinée à être utilisée dans la prévention ou le traitement d'une infection bactérienne exprimant un superantigène staphylococcique, et/ou un état pathologique médié directement ou indirectement par l'exposition à des toxines superantigéniques staphylococciques.

14. Préparation vaccinale destinée à être utilisée selon la revendication 13, un patient étant immunisé avec la préparation vaccinale, qui présente un risque de complications staphylococciques suite à une intervention chirurgicale ou au traitement invasif d'une maladie, de préférence l'état pathologique étant un état de septicémie induit par des médiateurs microbiens d'agents pathogènes ou de toxines bactériens à Gram positif, viraux ou fongiques, de préférence l'état de septicémie étant une septicémie, le syndrome de choc toxique (TSS) ou un choc septique.

15. Utilisation de la toxine SEB détoxifiée selon l'une quelconque des revendications 1 à 4, ou de l'antigène vaccinal selon l'une quelconque des revendications 5 à 7, dans une méthode in vitro de
a) production d'une préparation vaccinale, ou
b) production d'anticorps reconnaissant spécifiquement la toxine SEB.

16. Méthode de production de la toxine SEB détoxifiée selon l'une quelconque des revendications 1 à 4, ou de l'antigène vaccinal selon l'une quelconque des revendications 5 à 7, en exprimant une séquence d'acide nucléique codant la toxine ou l'antigène respectif dans une cellule hôte recombinante, et en isolant et en purifiant éventuellement la toxine ou l'antigène respectif.

17. Molécule d'acide nucléique codant la toxine SEB détoxifiée selon l'une quelconque des revendications 1 à 4, ou l'antigène vaccinal selon l'une quelconque des revendications 5 à 7, de préférence comprenant ou constitué de l'une quelconque des SEQ ID N° : 2, 4, 6, 8, 10, 12, 14, 32, 34 ou 36, ou de variants à codons optimisés de l'une quelconque des séquences précédentes.

18. Cellule hôte recombinante comprenant la molécule d'acide nucléique selon la revendication 17 dans une construction d'expression.
